# EUROPÄISCHE PATENTANMELDUNG

(11) **EP 4 494 663 A2**
(43) Veröffentlichungstag der Anmeldung: **22.01.2025**
(21) Anmeldenummer: 24208481.2
(22) Anmeldetag: 26.07.2022
(51) Int. Cl.: A61L 2/24

(54) **VERFAHREN ZUM EINSCHLEUSEN VON ARTIKELN AUS EINEM GEBINDE IN DIE PROZESSKAMMER EINES CONTAINMENTS UNTER ASEPTISCHEN BEDINGUNGEN UND SCHLEUSENANORDNUNG DAZU**

(62) Teilanmeldung aus: 22187101.5
(71) Anmelder: SKAN AG, 4123 Allschwil (CH)
(72) Erfinder: Carli, Julia Annette, 4053 Basel (CH); Leuenberger, Philipp, 4052 Basel (CH); Müller, Mathieu, 68510 Sierentz (FR); Rethoret, Christophe, 68500 Issenheim (FR); Schneidler, Tobias Jan, 6332 Hagendorn (CH); Tondera, Marc, 4125 Riehen (CH); Zeller, Mike, 4246 Wahlen (CH)
(74) Vertreter: Maucher Jenkins Patent- und Rechtsanwälte

(57) **Zusammenfassung**

Es wird ein Verfahren zum kontaminationsfreien Einschleusen eines sterilisierten Objektes aus einem mit einer Abdeckung (98) verschlossenen Behältnis (94) in eine von einer Wandung umgebenen Prozesskammer eines Containments vorgeschlagen, gekennzeichnet durch die folgenden Schritte: eine Passage des Containments wird einerseits von einem in der Prozesskammer herangeführten Deckel und andererseits von einem in der Eingangsstation herangeführten Gebinde mit dessen der Passage zugewandter Abdeckung (98) verschlossen, bei einem an die Passage herangeführtem Gebinde wird eine der Passage zugewandte Oberfläche der Abdeckung mittels einer Dekontaminationsvorrichtung dekontaminiert, nach erfolgter Dekontamination der der Passage zugewandten Oberfläche der Abdeckung (98), einschließlich deren Umfeld, wird die Abdeckung (98) geöffnet, um das Objekt durch die Passage in die Prozesskammer zu transferieren, nach einem Entleeren des Behältnisses wird der Deckel wieder an die Passage herangebracht, um anschließend das von den Objekten geleerte Behältnis von der Passage zu entfernen (Fig. 3A).

## Beschreibung

Die vorliegende Erfindung betrifft ein Verfahren und eine in einem Aufstellraum positionierte Schleusenanordnung zum Einschleusen von Artikeln unter aseptischen Bedingungen aus seriell zugeführten Gebinden in eine von einem Gehäuse umgebene Prozesskammer eines Containments. Der Prozesskammer ist eine Eingangsstation vorgelagert. Ein einzelnes Gebinde umfasst ein Behältnis mit einem aseptischen Innenraum und einem Durchlass, der mit einer Abdeckung verschlossen ist. Die im Innenraum gelagerten Artikel sollen nach dem Öffnen der Abdeckung in der Prozesskammer behandelt werden. Die Behältnisse der Gebinde haben die Gestalt wannenförmiger Tubs, und die Artikel sind z.B. pharmazeutische Phiolen, Vials oder Spritzen, die in systematisch angeordneten muldenförmigen Aufnahmekonturen von Nestern stecken.

### Stand der Technik

Aus der WO 2020/016 645 A2 ist eine Anordnung zum kontaminationsfreien Einschleusen eines sterilen Objektes aus einem Behältnis, welches mit einer semipermeablen Abdeckung verschlossen ist, in eine von einer Wandung umgebenen Arbeitskammer eines Containments bekannt. Die Anordnung umfasst zunächst eine Toreinheit mit einem in der Wandung angeordneten Zugangsflansch, der einen Durchgang von aussen in die Arbeitskammer ausbildet, und einer den Durchgang dicht verschliessenden Tür, die sich zur Offenstellung in die Arbeitskammer bewegen lässt. Zur Anordnung gehört ferner eine Behältnisaufnahme mit einem Auffang zur Halterung eines in die Behältnisaufnahme eingebrachten Behältnisses, einer Öffnung zum Einbringen des Behältnisses in den Auffang und einem Flansch zum Zusammenwirken mit dem Zugangsflansch.

Eine Dekontaminationseinheit ist dazu vorgesehen, bei geschlossener Tür, am Zugangsflansch angedockter Behältnisaufnahme und in der Behältnisaufnahme gelagertem Behältnis, eine der Tür zugewandte Aussenfläche der Abdeckung zu dekontaminieren. In Schliessstellung ist die Tür vonseiten der Arbeitskammer abgedichtet an den Zugangsflansch angefügt, und der Zugangsflansch umrandet einen Zwischenraum, der im Durchgang liegt. Die Abdeckung des in der Behältnisaufnahme eingestellten Behältnisses ist bei an den Zugangsflansch herangeschwenkter Behältnisaufnahme zwischen dem Flansch und dem Zugangsflansch abgedichtet. Die Dekontaminationseinheit ist mit Wirkungsrichtung in den Durchgang hinein direkt an der Tür oder seitlich der Toreinheit installiert, welche die Tür und den Zugangsflansch aufweist. Die Dekontaminationseinheit ist als Strahlungsquelle, z.B. UVC, UV, E-Beam oder Puls-Licht, oder als Begasungseinrichtung, z.B. zur Verneblung einer H2 O2 -Lösung ausgebildet.

### Aufgabe der Erfindung

Angesichts der beschränkten Produktivität der gemäss vorbekanntem Stand der Technik existierenden Vorrichtungen und praktizierten Verfahren, liegt der Erfindung die Aufgabe zugrunde, ein für das hiesige Anwendungsgebiet effizienteres Verfahren zum Einschleusen von Artikeln aus einem Gebinde in die Prozesskammer eines Containments unter aseptischen Bedingungen und eine dazu geeignete Schleusenanordnung vorzuschlagen. Massgeblich hierbei sind ein möglichst hoher Automatisierungsgrad, die weitgehende Vermeidung manuellen Eingreifens und Gewährleistung hoher Reinheitserfordernisse. Ausserdem ist darauf zu orientieren, dass bei möglichst geringem zeitlichem und apparativem Dekontaminationsaufwand möglichst nur Artikel in die Prozesskammer transferiert werden, die zu verarbeiten sind.

### Übersicht über die Erfindung

Das erfindungsgemässe Verfahren bzw. die erfindungsgemässe Schleusenanordnung ist zum Einschleusen von Artikeln unter aseptischen Bedingungen aus seriell zugeführten Gebinden in eine von einem Gehäuse umgebene Prozesskammer eines Containments mittels einer in einem Aufstellraum positionierten Schleusenanordnung konzipiert. Der Prozesskammer ist eine Eingangsstation vorgelagert. Ein einzelnes Gebinde umfasst ein Behältnis mit einem aseptischen Innenraum und einem Durchlass, der mit einer Abdeckung verschlossen ist, wobei die im Innenraum gelagerten Artikel nach dem Öffnen der Abdeckung in der Prozesskammer zu behandeln sind.

Im Produktionsmodus der Schleusenanordnung werden die aufeinanderfolgenden Verfahrensschritte ausgeführt:
- zum Transfer der Artikel aus den Gebinden in die Prozesskammer durch von der Eingangsstation sind zumindest zwei in die Prozesskammer führende Passagen vorgesehen und jede der Passagen wird einerseits von einem in der Prozesskammer herangeführten Deckel und/oder andererseits von einem in der Eingangsstation herangeführten Gebinde mit dessen der Passage zugewandten Abdeckung verschlossen;
- bei an die Passage herangeführtem Gebinde wird die der Passage zugewandte Oberfläche der Abdeckung mit eingeschlossenem Umfeld mittels einer Dekontaminationsvorrichtung dekontaminiert;
- nach erfolgter Dekontamination der der Passage zugewandten Oberfläche der Abdeckung, einschliesslich deren Umfeld, wird die Abdeckung geöffnet, um die Artikel aus dem Behältnis durch dessen Durchlass in die Prozesskammer zu transferieren; und
- nach Entleerung des Behältnisses wird zunächst der Deckel wieder an die betreffende Passage herangebracht, um anschliessend das von den Artikeln geleerte Behältnis von der Passage zu entfernen; wobei
- der Deckel an den Passagen im Wechsel zum Einsatz kommt.

Nachfolgend werden spezielle Ausführungsformen des erfindungsgemässen Verfahrens bzw. der Schleusenanordnung definiert:
Die Dekontaminationsvorrichtung ist im Deckel integriert oder ausserhalb des Deckels angeordnet, wobei das Emissionselement der Dekontaminationsvorrichtung direkt im Deckel oder ausserhalb des Deckels in einer Aufnahme sitzt.

Das eingeschlossene Umfeld umfasst das darin vorhandene Gasvolumen und vom Gasvolumen umspülte Oberflächen, nämlich Oberflächen:
- des Deckels;
- der optionalen Aufnahme;
- einer optionalen Dichtung; und
- von optional freiliegenden Wandungsflächen.

Die Prozesskammer ist mit einem Manipulator oder zusätzlich mit einem zweiten Manipulator ausgestattet, an deren schwenkbaren Auslegern jeweils ein mit Werkzeugen bestückter Werkzeugkopf sitzt, wobei:
- die Werkzeuge ausgebildet sind, um die Abdeckung zu öffnen und anschliessend die Artikel aus dem Behältnis durch den freigelegten Durchlass in die Prozesskammer zu transferieren; und
- der eine Manipulator mit dessen Werkzeugen wechselhaft an den Passagen zum Öffnen der Abdeckung und Transfer der Artikel in die Prozesskammer zum Einsatz kommt; oder
- der eine Manipulator und der zweite Manipulator jeweils nur an einer der Passagen oder die beiden Manipulatoren wechselhaft an den Passagen zum Einsatz kommen.

Ein Schneidwerkzeug ist Komponente der Werkzeuge, um für das Öffnen die Abdeckung aufzuschneiden. Vorzugsweise bestehen von der Abdeckung und der Umhüllung zumindest Flächenanteile aus einem semipermeablen Vliesgewebe, wie Tyvek^{®}. Die Abdeckung bewahrt vor dem Entfernen den Innenraum des Behältnisses in sterilem Zustand und ist auf einem den Durchlass des Behältnisses umlaufenden Rand versiegelt, z.B. durch Verkleben oder Verschweissen.

Im Behältnis lagert zur Aufnahme einer Vielzahl von Artikeln ein entnehmbares Nest, welches zusammen mit den Artikeln in die Prozesskammer transferiert wird. Nach Leerung des Nestes von den Artikeln in der Prozesskammer wird dieses geleerte Nest aus der Prozesskammer in ein nächstes oder dasselbe an der betreffenden Passage angedrücktes geleertes Behältnis entsorgt. Alternativ werden die Artikel direkt aus dem im Behältnis verbleibenden Nest in die Prozesskammer transferiert.

Die Dekontaminationsvorrichtung wird vorzugsweise als Strahlungsquelle, z.B. UVC, UV, E-Beam oder Puls-Licht, oder alternativ als eine Begasungseinrichtung, z.B. zur Verneblung einer H2 O2 -Lösung, ausgebildet.

Die von einem Gehäuse umgebene Eingangsstation weist einen zu einer angrenzenden Bereitstellungsstation führenden Zugang auf, wobei:
- die Eingangsstation mit einer gereinigten, gleichgerichteten Verdrängungsströmung betrieben wird, die teilweise durch den Zugang austritt;
- an der Bereitstellungsstation die Gebinde angeliefert werden, welche im Anlieferungszustand jeweils von einer Umhüllung umgeben sind, um das Innenvolumen des Gebindes mit dessen Inhalt aseptisch zu erhalten; und
- an der Bereitstellungsstation eine an der Umhüllung angebrachte Öffnung, die man bis anhin geschlossen hält, nun auf der der aus dem Zugang austretenden Verdrängungsströmung zugewandten Seite freigegeben wird, um das einzelne Gebinde in die Eingangsstation einzubringen; oder
- an der Bereitstellungsstation die Umhüllung auf der der aus dem Zugang austretenden Verdrängungsströmung zugewandten Seite aufgetrennt wird, um das einzelne Gebinde in die Eingangsstation einzubringen.

Die Positionierung des in der zuerst noch geschlossenen Umhüllung steckenden Gebindes erfolgt auf einer Auflage. Zum Ausschieben des Gebindes aus der offenen Umhüllung, bei auf das Gebinde fliessender Verdrängungsströmung, dient ein Vorschubmittel. Die Umhüllung wird von einem Greifer, vorzugsweise als Sauger ausgebildet, gehalten.

Die in die Eingangsstation eingebrachten Gebinde werden mittels zumindest einer Fördereinrichtung den Passagen zugeführt und nach der Verarbeitung über diese Fördereinrichtung wieder abgeführt. Die zumindest eine Fördereinrichtung beruht vorzugsweise auf dem Prinzip der Magnetschwebetechnik und erlaubt die Förderung auf mehreren Spuren, auch in gegensätzlicher Richtung.

Mittels einer ersten Fördereinrichtung erfolgt der Transport der Gebinde innerhalb der Eingangsstation, während eine zweite Fördereinrichtung das Heranschwenken der Gebinde mit an der jeweiligen Passage angepressten Abdeckung bewirkt. Die zweite Fördereinrichtung ist auch zum Entfernen bearbeiteter Gebinde von der jeweiligen Passage nutzbar. Zur Positionierung der Gebinde an der jeweiligen Passage umfasst die zweite Fördereinrichtung vorzugsweise ein kardanisches Lager.

Die zweite Fördereinrichtung besteht aus:
- einer Achse, durch welche sich eine erste Drehachse erstreckt;
- zwei nebeneinander an der Achse fixierten Gabeln, die jeweils um eine zweite Drehachse schwenkbar sind, welche sich rechtwinklig zur ersten Drehachse erstrecken; und
- jeweils einen in der zugehörigen Gabel aufgehängten und um eine dritte Drehachse schwenkbaren Träger, wobei die erste Drehachse und die dritte Drehachse zueinander parallel verlaufen und die Träger zum Unterfassen der Ränder der Behältnisse beim An- bzw. Abschwenken von den Passagen konfiguriert sind.

Zwei nebeneinander liegende kardanische Lager sind jeweils gebildet aus einen U-förmigen Träger, der in der dritten Drehachse jeweils mit einer Gabel verbunden ist, die in der zweiten Drehachse aufgehängt ist.

In jeder Passage sitzt eine Dichtung, welche zusammen mit der Abdeckung eines temporär angepressten Gebindes und/oder mit einem temporär angepressten Deckel an den Passagen, im Übergang von der Eingangsstation zur Prozesskammer, Abdichtungen schafft. Anstelle einer herkömmlichen Dichtung kann man einen minimen Spalt vorsehen oder eine LabyrinthDichtung einsetzen. Der Deckel wird von einem schwenkbaren Ausleger einer in der Prozesskammer positionierten Apparatur getragen.

Der zumindest eine Manipulator oder auch der zusätzliche Manipulator sind vorzugsweise als Roboter ausgebildet.

Die Behältnisse der Gebinde haben die Gestalt wannenförmiger Tubs. Die Artikel sind z.B. Phiolen, Vials oder Spritzen, die in systematisch angeordneten Aufnahmekonturen von Nestern stecken.

Bei einer ersten bevorzugten Ausgestaltung des erfindungsgemäßen Verfahrens, beispielsweise bei einem Gegenstand nach einem der nachstehenden Ansprüche, kann vorgesehen sein, dass a) die Dekontaminationsvorrichtung im Deckel integriert oder ausserhalb des Deckels angeordnet ist; und b) das Emissionselement der Dekontaminationsvorrichtung direkt im Deckel oder ausserhalb des Deckels 51 in einer Aufnahme angeordnet ist.

Bei einer zweiten bevorzugten Ausgestaltung des erfindungsgemäßen Verfahrens, beispielsweise bei einem Gegenstand nach einem der nachstehenden Ansprüche, kann alternativ oder zusätzlich zur ersten bevorzugten Ausgestaltung vorgesehen sein, dass das eingeschlossene Umfeld das darin vorhandene Gasvolumen und vom Gasvolumen umspülte Oberflächen umfasst, nämlich Oberflächen: a) des Deckels; b) der optionalen Aufnahme; c) einer optionalen Dichtung; und d) von optional freiliegenden Wandungsflächen.

Bei einer dritten bevorzugten Ausgestaltung des erfindungsgemäßen Verfahrens, beispielsweise bei einem Gegenstand nach einem der nachstehenden Ansprüche, kann alternativ oder zusätzlich zur ersten oder zweiten bevorzugten Ausgestaltung vorgesehen sein, dass a) die Prozesskammer mit einem Manipulator oder zusätzlich mit einem zweiten Manipulator ausgestattet ist, an deren schwenkbaren Auslegern jeweils ein mit Werkzeugen bestückter Werkzeugkopf sitzt, wobei: b) die Werkzeuge ausgebildet sind, um die Abdeckung zu öffnen und anschliessend die Artikel aus dem Behältnis durch den freigelegten Durchlass in die Prozesskammer zu transferieren; und c) der eine Manipulator mit dessen Werkzeugen wechselhaft an den Passagen zum Öffnen der Abdeckung und Transfer der Artikel in die Prozesskammer zum Einsatz kommt; oder d) der eine Manipulator und der zweite Manipulator jeweils nur an einer der Passagen oder die beiden Manipulatoren wechselhaft an den Passagen zum Einsatz kommen.

Bei einer vierten bevorzugten Ausgestaltung des erfindungsgemäßen Verfahrens kann alternativ oder zusätzlich zur ersten bis dritten bevorzugten Ausgestaltung vorgesehen sein, dass a) ein Schneidwerkzeug Komponente der Werkzeuge ist, um für das Öffnen die Abdeckung aufzuschneiden; wobei b) vorzugsweise von der Abdeckung und der Umhüllung zumindest Flächenanteile aus einem semipermeablen Vliesgewebe, wie Tyvek ^{®} bestehen, und die Abdeckung vor dem Entfernen den Innenraum des Behältnisses in sterilem Zustand bewahrt und auf einem den Durchlass des Behältnisses umlaufenden Rand versiegelt ist.

Bei einer fünften bevorzugten Ausgestaltung des erfindungsgemäßen Verfahrens kann alternativ oder zusätzlich zur ersten bis vierten bevorzugten Ausgestaltung vorgesehen sein, dass a) im Behältnis zur Aufnahme einer Vielzahl von Artikeln ein entnehmbares Nest lagert, welches zusammen mit den Artikeln in die Prozesskammer transferiert wird; und b) nach Leerung des Nestes von den Artikeln in der Prozesskammer, dieses geleerte Nest aus der Prozesskammer in ein nächstes oder dasselbe an der betreffenden Passage angedrücktes geleertes Behältnis entsorgt wird; oder c) die Artikel direkt aus dem im Behältnis verbleibenden Nest in die Prozesskammer transferiert werden.

Bei einer sechsten bevorzugten Ausgestaltung des erfindungsgemäßen Verfahrens kann alternativ oder zusätzlich zur ersten bis fünften bevorzugten Anwendung vorgesehen sein, dass die Dekontaminationsvorrichtung vorzugsweise als Strahlungsquelle, z.B. UVC, UV, E-Beam oder Puls-Licht, oder alternativ als eine Begasungseinrichtung, z.B. zur Verneblung einer H 2 O 2 -Lösung, ausgebildet wird.

Bei einer siebten bevorzugten Anwendung der Erfindung kann alternativ oder zusätzlich zur ersten bis sechsten bevorzugten Ausgestaltung vorgesehen sein, dass der Eingangsstation, welche von einem Gehäuse umgeben ist, das einen zu einer angrenzenden Bereitstellungsstation führenden Zugang aufweist, wobei: a) die Eingangsstation mit einer gereinigten, gleichgerichteten Verdrängungsströmung LF betrieben wird, die teilweise durch den Zugang austritt; b) an der Bereitstellungsstation die Gebinde angeliefert werden, welche im Anlieferungszustand jeweils von einer Umhüllung umgeben sind, um das Innenvolumen des Gebindes mit dessen Inhalt aseptisch zu erhalten; und c) an der Bereitstellungsstation eine an der Umhüllung angebrachte Öffnung, die man bis anhin geschlossen hält, nun auf der der aus dem Zugang austretenden Verdrängungsströmung zugewandten Seite freigegeben wird, um das einzelne Gebinde in die Eingangsstation einzubringen; oder d) an der Bereitstellungsstation die Umhüllung auf der der aus dem Zugang austretenden Verdrängungsströmung zugewandten Seite aufgetrennt wird, um das einzelne Gebinde in die Eingangsstation einzubringen.

Bei einer achten bevorzugten Ausgestaltung des erfindungsgemäßen Verfahrens kann zusätzlich zur siebten bevorzugten Ausgestaltung vorgesehen sein, dass a) die Positionierung des in der zuerst noch geschlossenen Umhüllung steckenden Gebindes auf einer Auflage erfolgt; b) zum Ausschieben des Gebindes aus der offenen Umhüllung, bei auf das Gebinde fliessender Verdrängungsströmung, ein Vorschubmittel dient; und c) die Umhüllung von einem Greifer, vorzugsweise als Sauger ausgebildet, gehalten wird.

Bei einer neunten bevorzugten Ausgestaltung des erfindungsgemäßen Verfahrens kann alternativ oder zusätzlich zur ersten bis achten bevorzugten Ausgestaltung vorgesehen sein, dass a) die in die Eingangsstation eingebrachten Gebinde mittels zumindest einer Fördereinrichtung den Passagen zugeführt werden; und b) die zumindest eine Fördereinrichtung vorzugsweise auf dem Prinzip der Magnetschwebetechnik beruht.

Bei einer zehnten bevorzugten Ausgestaltung des erfindungsgemäßen Verfahrens kann zusätzlich zur neunten bevorzugten Ausgestaltung vorgesehen sein, dass a) mittels einer ersten Fördereinrichtung der Transport der Gebinde innerhalb der Eingangsstation erfolgt; während b) eine zweite Fördereinrichtung das Heranschwenken der Gebinde mit an der jeweiligen Passage angepressten Abdeckung bewirkt; und c) die zweite Fördereinrichtung auch zum Entfernen bearbeiteter Gebinde von der jeweiligen Passage nutzbar ist.

Bei einer elften bevorzugten Ausgestaltung des erfindungsgemäßen Verfahrens kann zusätzlich zur zehnten bevorzugten Ausgestaltung vorgesehen sein, dass zur Positionierung der Gebinde an der jeweiligen Passage die zweite Fördereinrichtung ein kardanisches Lager umfasst.

Bei einer zwölften bevorzugten Ausgestaltung des erfindungsgemäßen Verfahrens kann alternativ oder zusätzlich zur zehnten oder elften bevorzugten Ausgestaltung vorgesehen sein, dass die zweite Fördereinrichtung besteht aus: a) einer Achse, durch welche sich eine erste Drehachse erstreckt; b) zwei nebeneinander an der Achse fixierten Gabeln, die jeweils um eine zweite Drehachse schwenkbar sind, welche sich rechtwinklig zur ersten Drehachse erstrecken; und c) jeweils einen in der zugehörigen Gabel aufgehängten und um eine dritte Drehachse schwenkbaren Träger, wobei die erste Drehachse und die dritte Drehachse zueinander parallel verlaufen und die Träger zum Unterfassen der Ränder der Behältnisse beim An- bzw. Abschwenken von den Passagen konfiguriert sind; wobei d) zwei nebeneinander liegende kardanische Lager jeweils gebildet sind aus einen U-förmigen Träger, der in der dritten Drehachse jeweils mit einer Gabel verbunden ist, die in der zweiten Drehachse aufgehängt ist.

Bei einer dreizehnten bevorzugten Ausgestaltung des erfindungsgemäßen Verfahrens kann alternativ oder zusätzlich zur ersten bis zwölften bevorzugten Anwendung vorgesehen sein, dass in jeder Passage eine Dichtung sitzt, welche zusammen mit der Abdeckung eines temporär angepressten Gebindes und/oder mit einem temporär angepressten Deckel an den Passagen, im Übergang von der Eingangsstation zur Prozesskammer, Abdichtungen schafft.

Bei einer vierzehnten bevorzugten Ausgestaltung des erfindungsgemäßen Verfahrens kann alternativ oder zusätzlich zur ersten bis dreizehnten bevorzugten Anwendung vorgesehen sein, dass der Deckel von einem schwenkbaren Ausleger einer in der Prozesskammer positionierten Apparatur getragen wird.

Bei einer fünfzehnten bevorzugten Anwendung der Erfindung kann alternativ oder zusätzlich zu den bevorzugten Anwendungen drei bis vierzehn vorgesehen sein, dass der zumindest eine Manipulator oder auch der zusätzliche Manipulator als Roboter ausgebildet sind.

Bei einer sechzehnten bevorzugten Ausgestaltung des erfindungsgemäßen Verfahrens kann alternativ oder zusätzlich zur ersten bis fünfzehnten bevorzugten Anwendung vorgesehen sein, dass die Behältnisse der Gebinde die Gestalt wannenförmiger Tubs haben und die Artikel, z.B. Phiolen, Vials oder Spritzen sind, die in systematisch angeordneten Aufnahmekonturen von Nestern stecken.

Bei einer ersten bevorzugten Ausgestaltung der Schleusenanordnung kann alternativ oder zusätzlich, insbesondere bei einem Gegenstand der nachstehenden Ansprüche, vorgesehen sein, dass a) die Dekontaminationsvorrichtung im Deckel integriert oder ausserhalb des Deckels angeordnet ist; und b) das Emissionselement der Dekontaminationsvorrichtung direkt im Deckel oder ausserhalb des Deckels in einer Aufnahme angeordnet ist.

Bei einer zweiten bevorzugten Ausgestaltung der Schleusenanordnung kann alternativ oder zusätzlich zur ersten bevorzugten Ausgestaltung vorgesehen sein, dass das eingeschlossene Umfeld 8 das darin vorhandene Gasvolumen und vom Gasvolumen umspülte Oberflächen umfasst, nämlich Oberflächen: a) des Deckels; b) der optionalen Aufnahme; c) einer optionalen Dichtung; und d) von optional freiliegenden Wandungsflächen.

Bei einer dritten bevorzugten Ausgestaltung der Schleusenanordnung kann alternativ oder zusätzlich zur ersten oder zweiten bevorzugten Ausgestaltung vorgesehen sein, dass a) die Prozesskammer mit einem Manipulator oder zusätzlich mit einem zweiten Manipulator ausgestattet ist, an deren schwenkbaren Auslegern jeweils ein mit Werkzeugen bestückter Werkzeugkopf sitzt, wobei: b) die Werkzeuge ausgebildet sind, um die Abdeckung zu öffnen und anschliessend die Artikel aus dem Behältnis durch den freigelegten Durchlass in die Prozesskammer zu transferieren; und c) der eine Manipulator mit dessen Werkzeugen wechselhaft an den Passagen zum Öffnen der Abdeckung und Transfer der Artikel in die Prozesskammer zum Einsatz kommt; oder d) der eine Manipulator und der zweite Manipulator jeweils nur an einer der Passagen oder die beiden Manipulatoren 6 wechselhaft an den Passagen zum Einsatz kommen.

Bei einer vierten bevorzugten Ausgestaltung der Schleusenanordnung kann alternativ oder zusätzlich zur ersten bis dritten bevorzugten Ausgestaltung vorgesehen sein, dass a) die Werkzeuge ein Schneidwerkzeug als Komponente aufweisen, bestimmt zum Öffnen der Abdeckung; wobei b) vorzugsweise von der Abdeckung und der Umhüllung zumindest Flächenanteile aus einem semipermeablen Vliesgewebe, wie Tyvek^{®} bestehen, und die Abdeckung vor dem Entfernen den Innenraum des Behältnisses in sterilem Zustand bewahrt und auf einem den Durchlass des Behältnisses umlaufenden Rand versiegelt ist.

Bei einer fünften bevorzugten Ausgestaltung der Schleusenanordnung kann alternativ oder zusätzlich zur ersten bis vierten bevorzugten Ausgestaltung vorgesehen sein, dass a) im Behältnis zur Aufnahme einer Vielzahl von Artikeln ein entnehmbares Nest lagert, welches zusammen mit den Artikeln in die Prozesskammer transferierbar ist; und b) das von Artikeln geleerte Nest aus der Prozesskammer in ein nächstes oder dasselbe an der betreffenden Passage angedocktes geleertes Behältnis entsorgbar ist; oder c) die Artikel direkt aus dem im Behältnis verbleibenden Nest in die Prozesskammer transferierbar sind.

Bei einer sechsten bevorzugten Ausgestaltung der Schleusenanordnung kann alternativ oder zusätzlich zur ersten bis fünften bevorzugten Ausgestaltung vorgesehen sein, dass die Dekontaminationsvorrichtung vorzugsweise als Strahlungsquelle, z.B. UVC, UV, E-Beam oder Puls-Licht, oder alternativ als eine Begasungseinrichtung, z.B. zur Verneblung einer H 2 O 2 -Lösung, ausgebildet ist.

Bei einer siebten bevorzugten Ausgestaltung der Schleusenanordnung kann alternativ oder zusätzlich zur ersten bis sechsten bevorzugten Ausgestaltung vorgesehen sein, dass der Eingangsstation, welche von einem Gehäuse umgeben ist, das einen zu einer angrenzenden Bereitstellungsstation führenden Zugang aufweist, wobei: a) die Eingangsstation mit einer gereinigten, gleichgerichteten Verdrängungsströmung LF betreibbar ist, die teilweise durch den Zugang austritt; b) die Bereitstellungsstation zur Anlieferung der Gebinde bestimmt ist, welche im Anlieferungszustand jeweils von einer Umhüllung umgeben sind, um das Innenvolumen des Gebindes mit dessen Inhalt aseptisch zu erhalten; und c) an der Bereitstellungsstation eine an der Umhüllung angebrachte Öffnung, die bis anhin mittels eines angesetzten Werkzeugs geschlossen bleibt, nun auf der der aus dem Zugang austretenden Verdrängungsströmung zugewandten Seite durch Lösen des Werkzeugs freikommt, so dass das einzelne Gebinde in die Eingangsstation einbringbar ist; oder d) an der Bereitstellungsstation die Umhüllung auf der der aus dem Zugang austretenden Verdrängungsströmung zugewandten Seite mittels eines Werkzeugs auftrennbar ist, so dass das einzelne Gebinde in die Eingangsstation einbringbar ist.

Bei einer achten bevorzugten Ausgestaltung der Schleusenanordnung kann zusätzlich zur siebten bevorzugten Ausgestaltung vorgesehen sein, dass a) zur Positionierung des in der zuerst noch geschlossenen Umhüllung steckenden Gebindes eine Auflage vorgesehen ist; b) zum Ausschieben des Gebindes aus der offenen Umhüllung, bei auf das Gebinde fliessender Verdrängungsströmung, ein Vorschubmittel dient; und c) zum Zurückhalten der Umhüllung ein Greifer, vorzugsweise als Sauger ausgebildet, dient.

Bei einer neunten bevorzugten Ausgestaltung der Schleusenanordnung kann alternativ oder zusätzlich zur ersten bis achten bevorzugten Ausgestaltung vorgesehen sein, dass a) zumindest eine Fördereinrichtung zur Zuführung der in die Eingangsstation eingebrachten Gebinde an die Passagen vorgesehen ist; und b) die zumindest eine Fördereinrichtung auf dem Prinzip der Magnetschwebetechnik beruht.

Bei einer zehnten bevorzugten Ausgestaltung der Schleusenanordnung kann zusätzlich zur neunten bevorzugten Ausgestaltung vorgesehen sein, dass a) zum Transport der Gebinde innerhalb der Eingangsstation eine erste Fördereinrichtung dient; während b) eine zweite Fördereinrichtung zum Heranschwenken der Gebinde mit an der jeweiligen Passage angepressten Abdeckung bestimmt ist; und c) die zweite Fördereinrichtung auch zum Entfernen bearbeiteter Gebinde von der jeweiligen Passage nutzbar ist.

Bei einer elften bevorzugten Ausgestaltung der Schleusenanordnung kann zusätzlich zur zehnten bevorzugten Ausgestaltung vorgesehen sein, dass zur Positionierung der Gebinde an der jeweiligen Passage die zweite Fördereinrichtung ein kardanisches Lager umfasst.

Bei einer zwölften bevorzugten Ausgestaltung der Schleusenanordnung kann alternativ oder zusätzlich zur zehnten oder elften bevorzugten Ausgestaltung vorgesehen sein, dass die zweite Fördereinrichtung besteht aus: a) einer Achse, durch welche sich eine erste Drehachse erstreckt; b) zwei nebeneinander an der Achse fixierten Gabeln, die jeweils um eine zweite Drehachse schwenkbar sind, welche sich rechtwinklig zur ersten Drehachse erstrecken; und c) jeweils einen in der zugehörigen Gabel aufgehängten und um eine dritte Drehachse schwenkbaren Träger, wobei die erste Drehachse und die dritte Drehachse zueinander parallel verlaufen und die Träger zum Unterfassen der Ränder der Behältnisse beim An- bzw. Abschwenken von den Passagen konfiguriert sind; wobei d) zwei nebeneinander liegende kardanische Lager jeweils gebildet sind aus einen U-förmigen Träger, der in der dritten Drehachse jeweils mit einer Gabel verbunden ist, die in der zweiten Drehachse aufgehängt ist.

Bei einer dreizehnten bevorzugten Ausgestaltung der Schleusenanordnung kann alternativ oder zusätzlich zur ersten bis zwölften bevorzugten Ausgestaltung vorgesehen sein, dass in jeder Passage eine Dichtung sitzt, welche zusammen mit der Abdeckung eines temporär angepressten Gebindes und/oder mit einem temporär angepressten Deckel an den Passagen, im Übergang von der Eingangsstation zur Prozesskammer, Abdichtungen schafft.

Bei einer vierzehnten bevorzugten Ausgestaltung der Schleusenanordnung kann alternativ oder zusätzlich zur ersten bis dreizehnten bevorzugten Ausgestaltung vorgesehen sein, dass ein schwenkbarer Ausleger einer in der Prozesskammer positionierten Apparatur den Deckel trägt.

Bei einer fünfzehnten bevorzugten Ausgestaltung der Schleusenanordnung kann alternativ oder zusätzlich zur dritten bis vierzehnten bevorzugten Ausgestaltung vorgesehen sein, der zumindest eine Manipulator oder auch der zusätzliche Manipulator als Roboter ausgebildet sind.

Bei einer sechzehnten bevorzugten Ausgestaltung der Schleusenanordnung kann alternativ oder zusätzlich zur ersten bis fünfzehnten bevorzugten Ausgestaltung vorgesehen sein, dass die Behältnisse der Gebinde die Gestalt wannenförmiger Tubs haben und die Artikel, z.B. Phiolen, Vials oder Spritzen sind, die in systematisch angeordneten Aufnahmekonturen von Nestern stecken.

### Kurzbeschreibung der beigefügten Zeichnungen

Es zeigen:

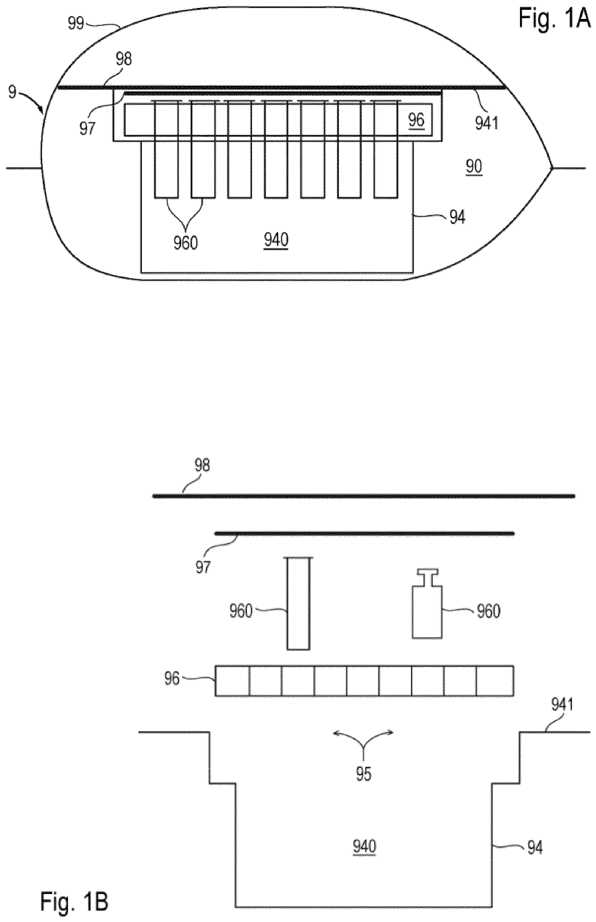

Figur 1A - ein mit dem erfindungsgemässen Verfahren in der zugehörigen Schleusenanordnung zu verarbeitendes Gebinde mit geschlossener Umhüllung;
Figur 1B - das Gebinde gemäss Figur 1A ohne die Umhüllung in Explosivansicht;

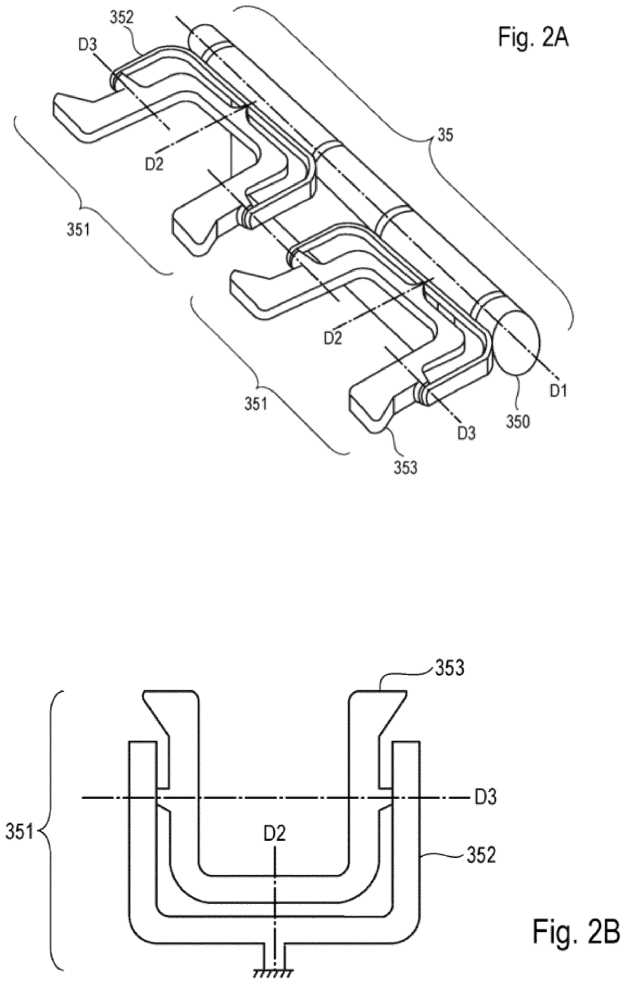

Figur 2A - die zweite Fördereinrichtung als Bestandteil der Schleusenanordnung, bestehend aus der Achse und daran fixierten zwei kardanischen Lagern, in Perspektivansicht;
Figur 2B - ein kardanisches Lager aus Figur 2A, in Draufsicht;
Figuren 3A bis 15D - die Gesamtheit eines Verfahrensdurchlaufs mit jeweils vier Figuren der Schleusenanordnung, nämlich deren vordere Sektion gemäss Figuren A, hintere Sektion gemäss Figuren B, in Draufsicht gemäss Figuren C und als prinzipielle Seitenansicht gemäss Figuren D;

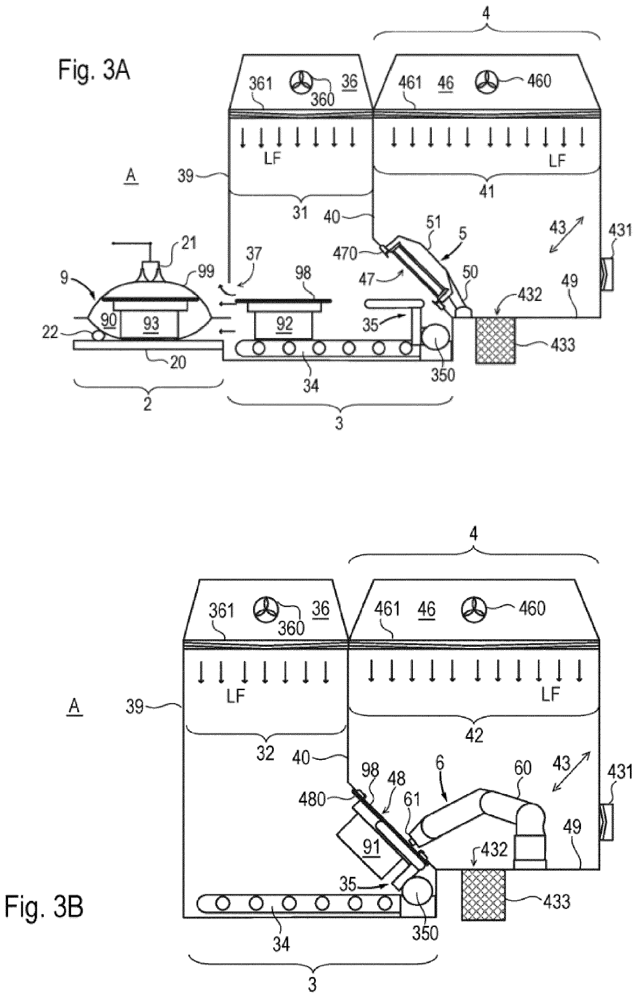

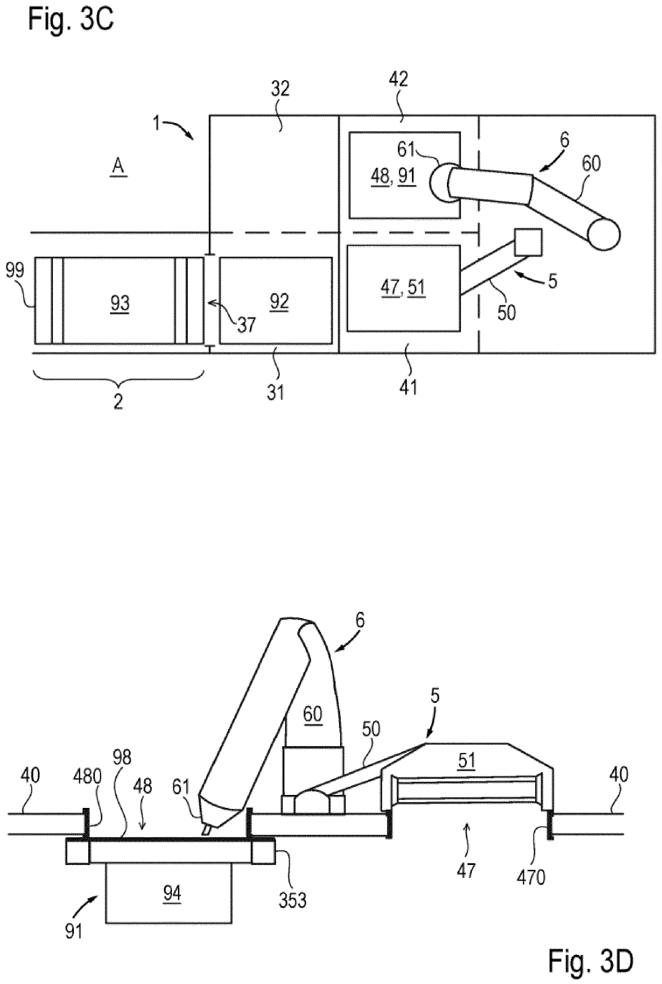

Figuren 3A bis 3D - Prozessphase 1 (Startposition)

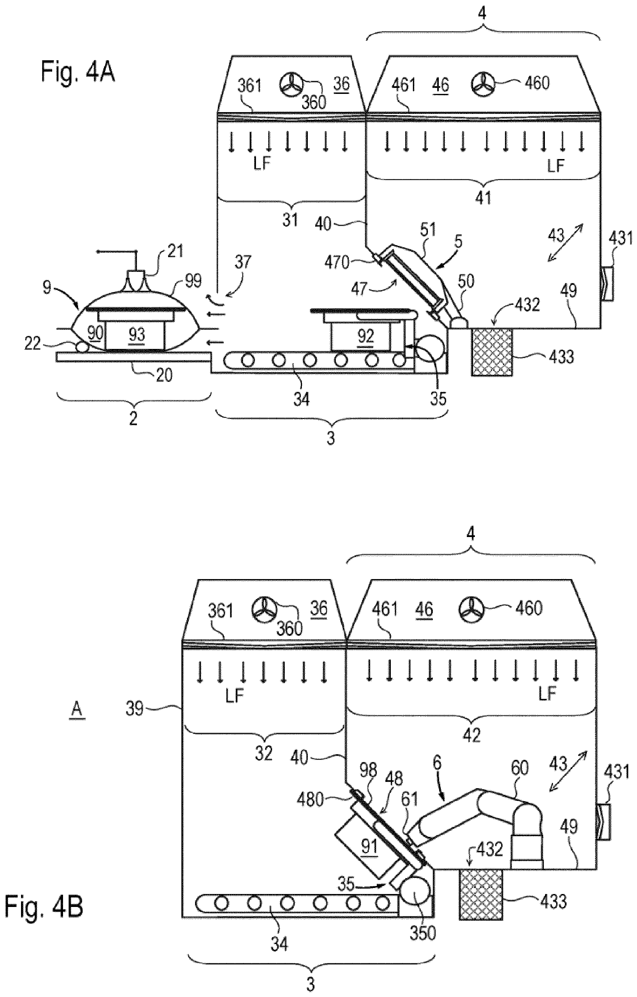

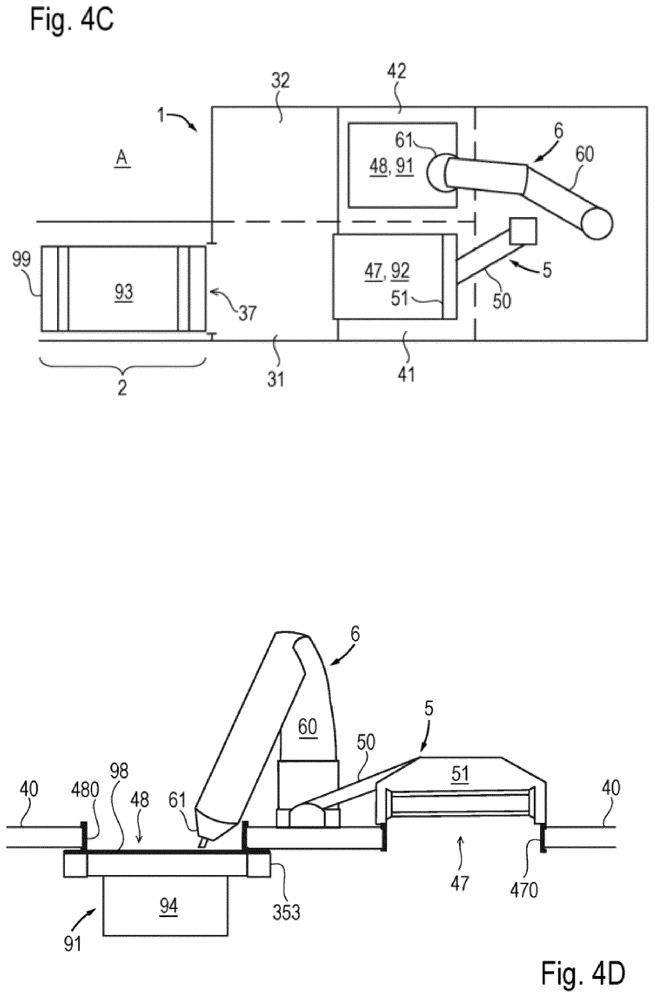

Figuren 4A bis 4D - Prozessphase 2

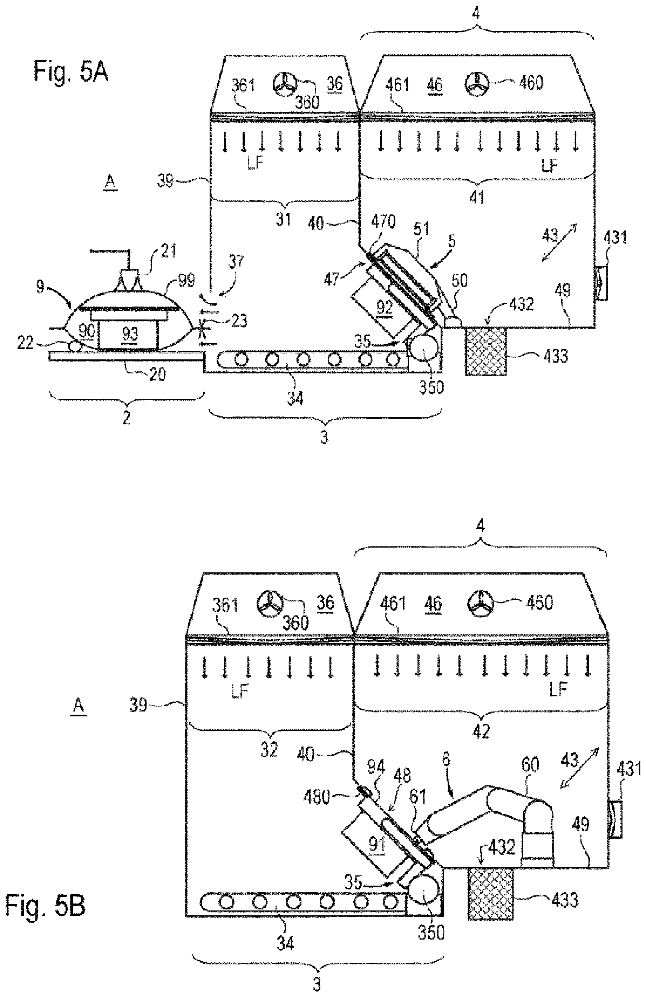

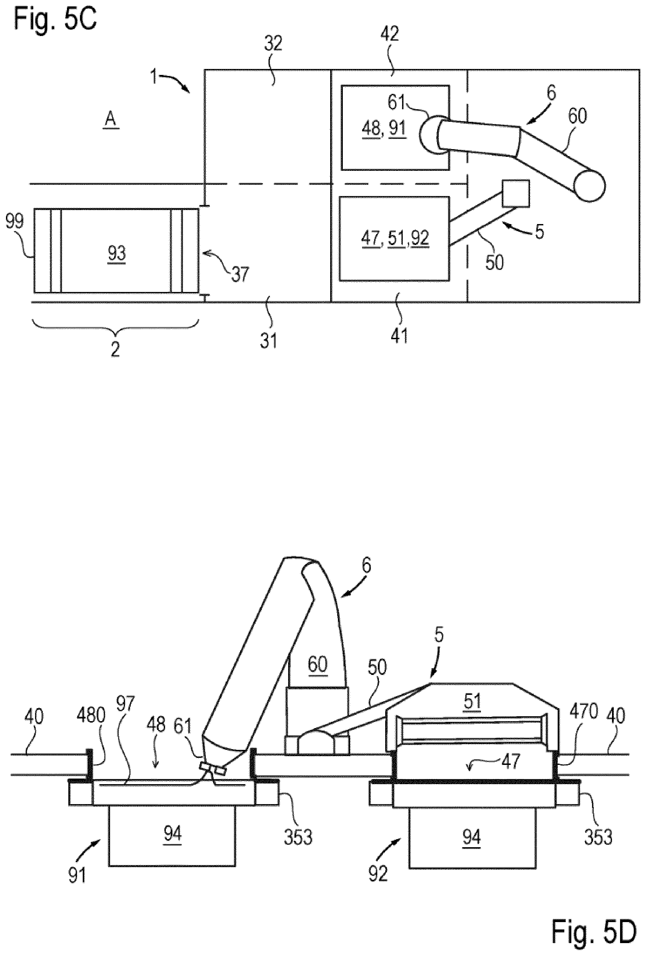

Figuren 5A bis 5D - Prozessphase 3

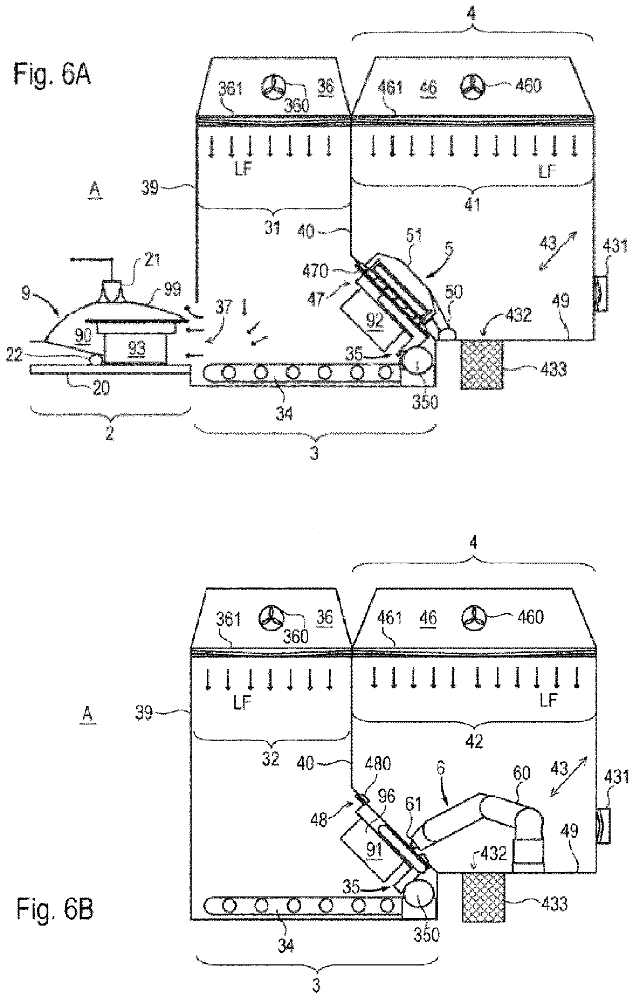

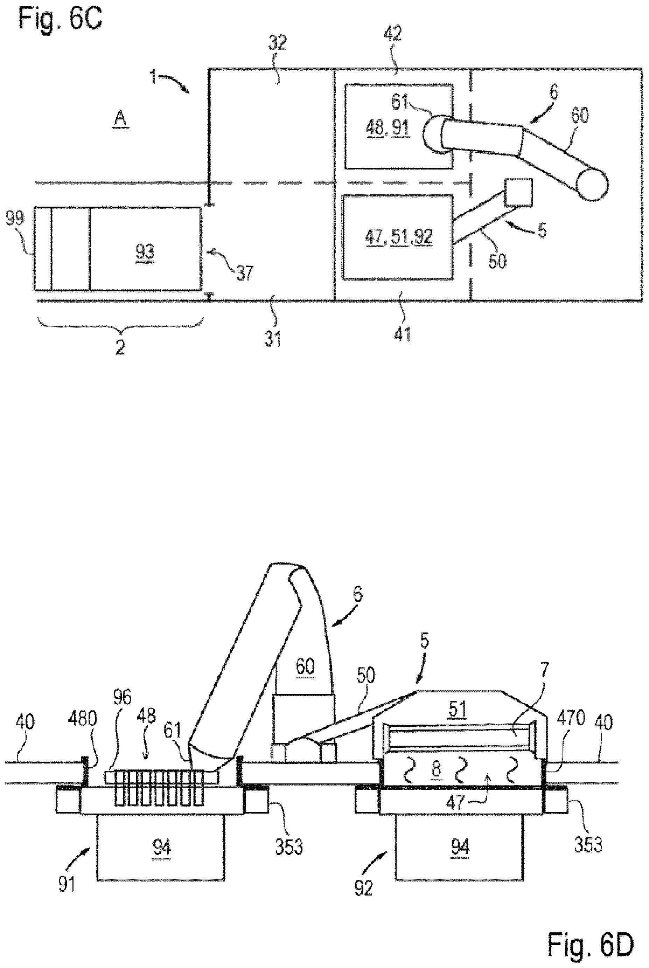

Figuren 6A bis 6D - Prozessphase 4

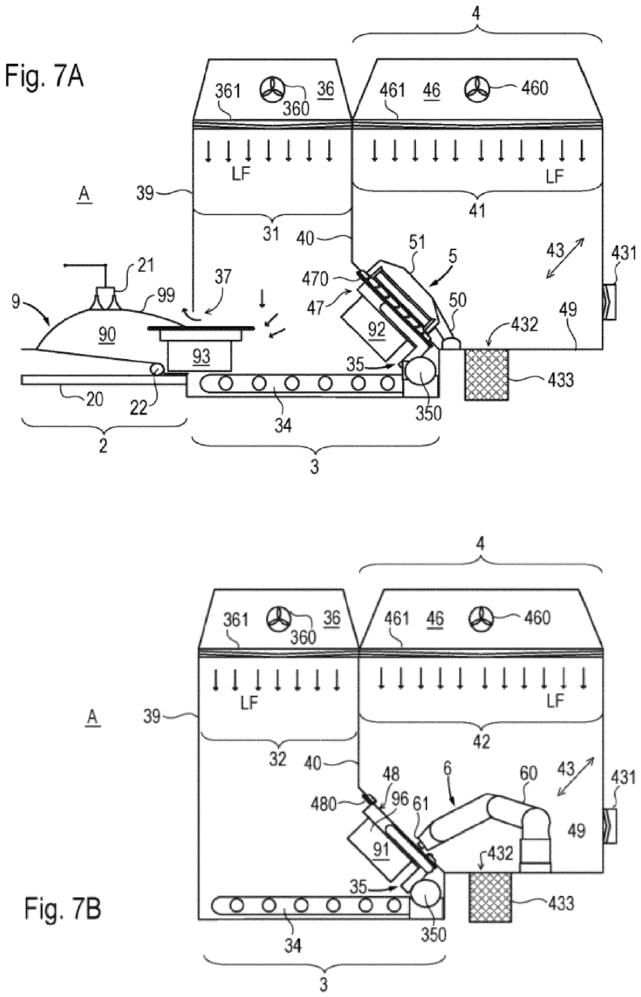

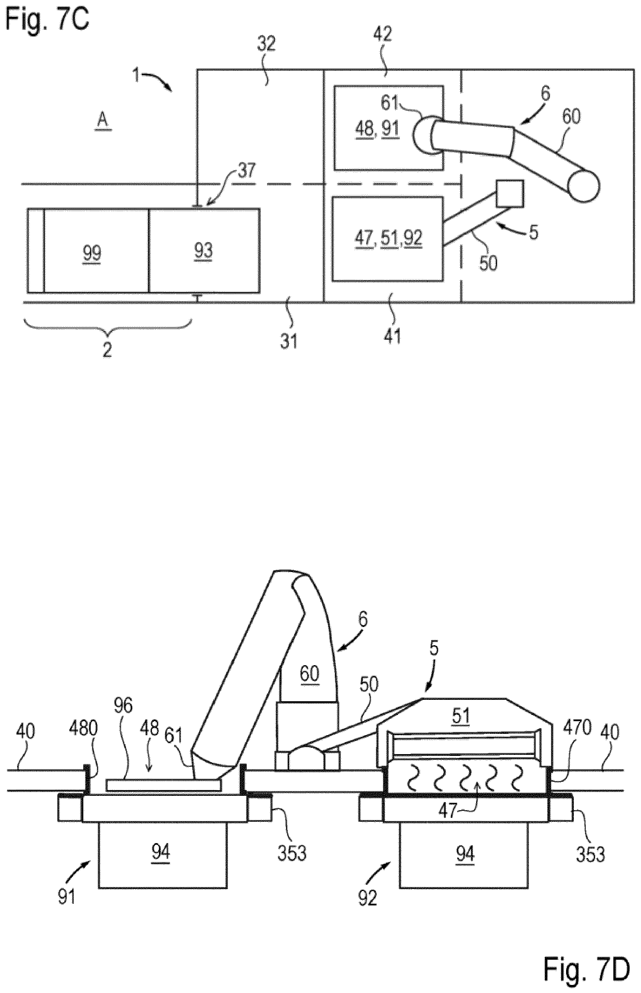

Figuren 7A bis 7D - Prozessphase 5

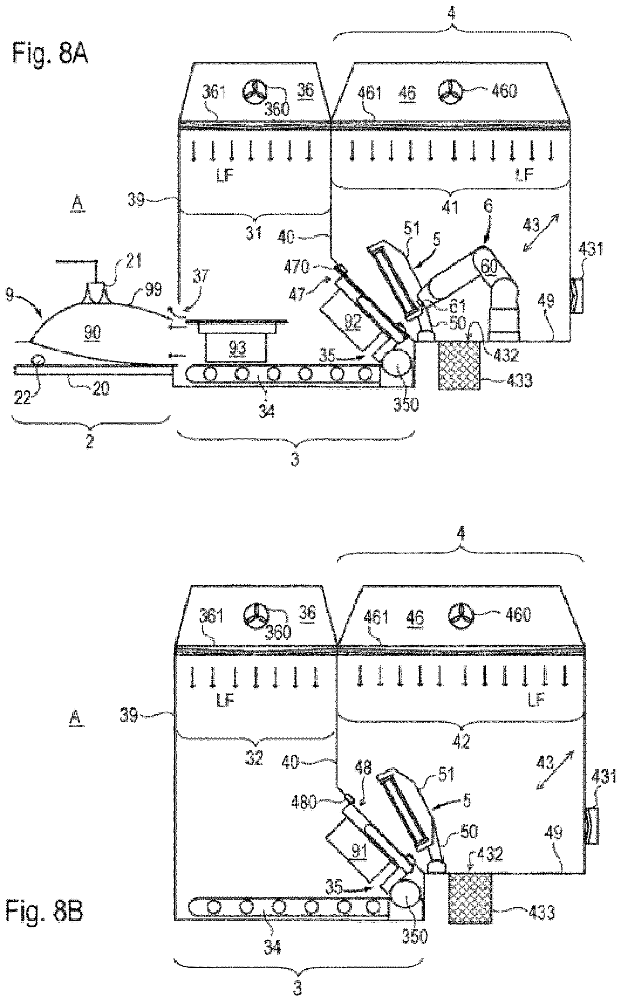

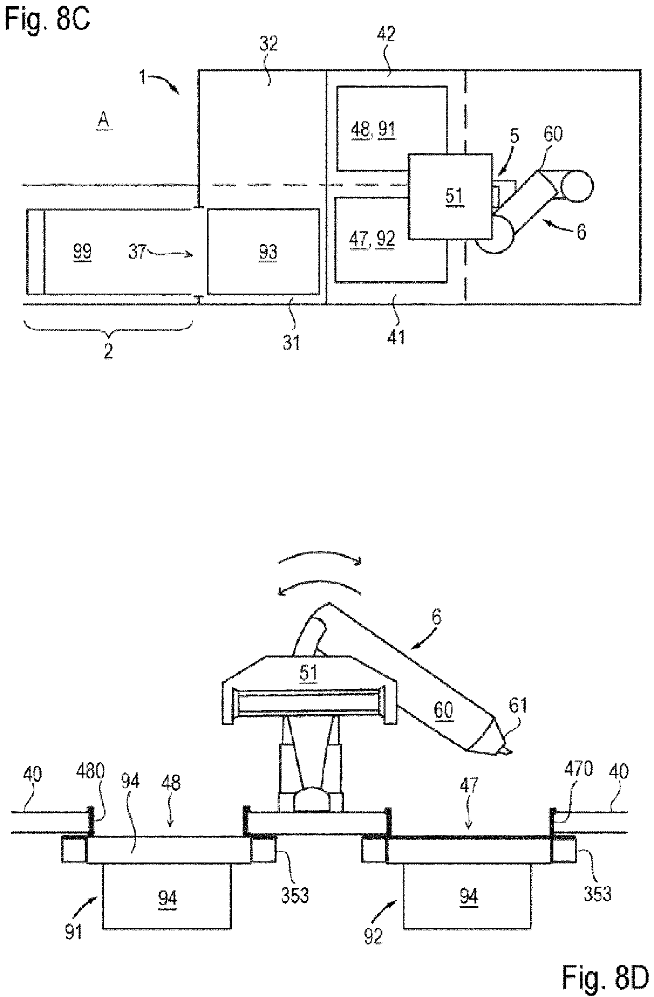

Figuren 8A bis 8D - Prozessphase 6

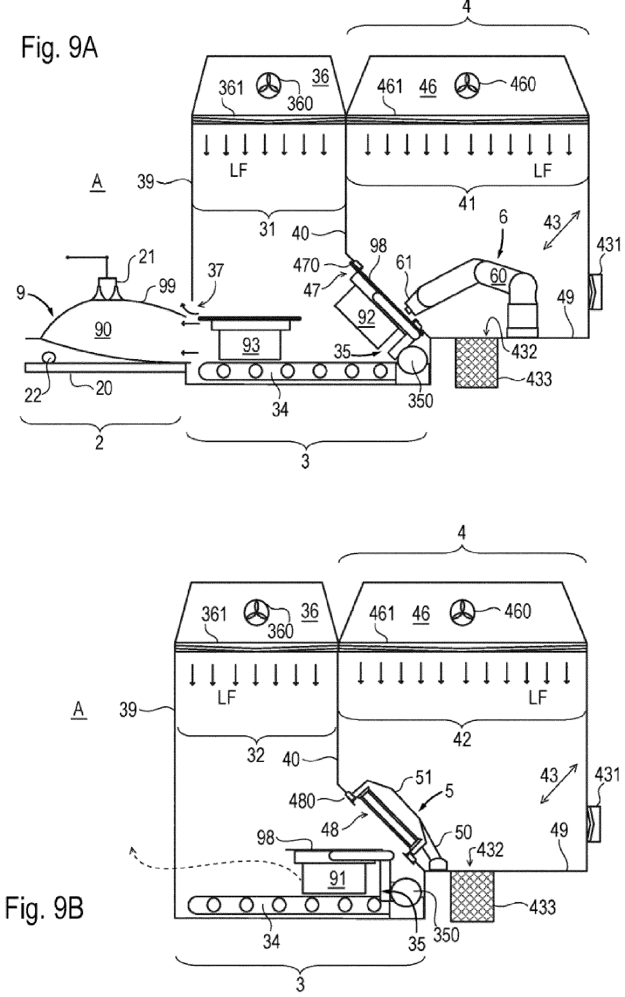

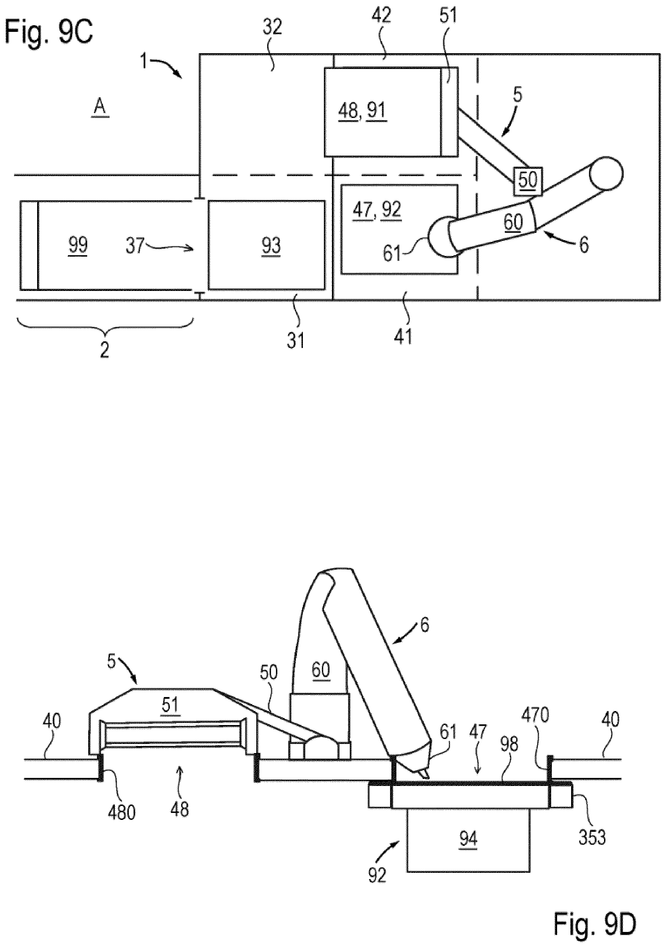

Figuren 9A bis 9D - Prozessphase 7

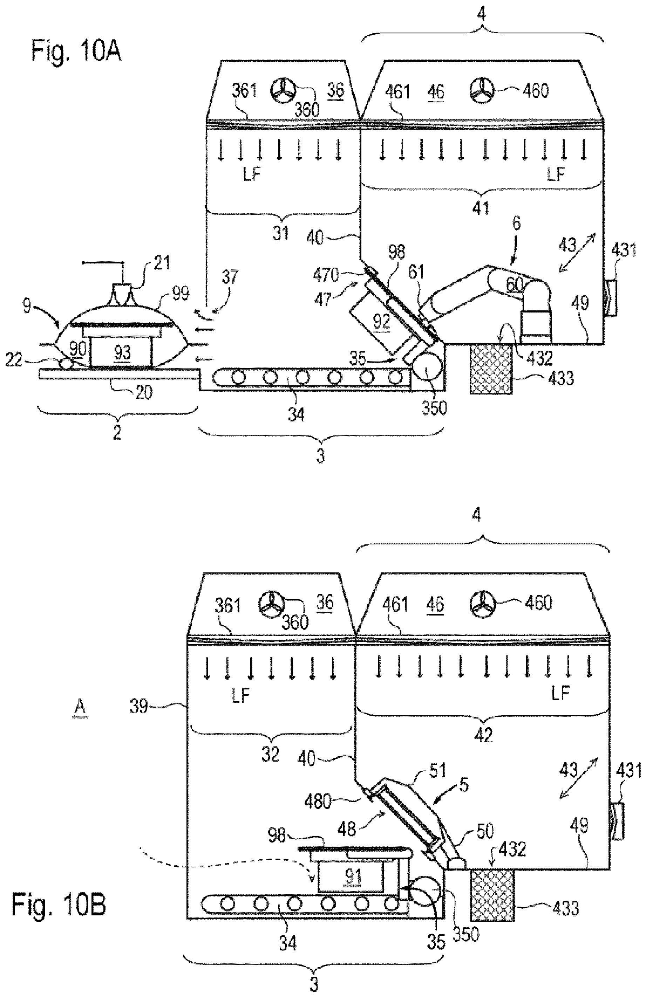

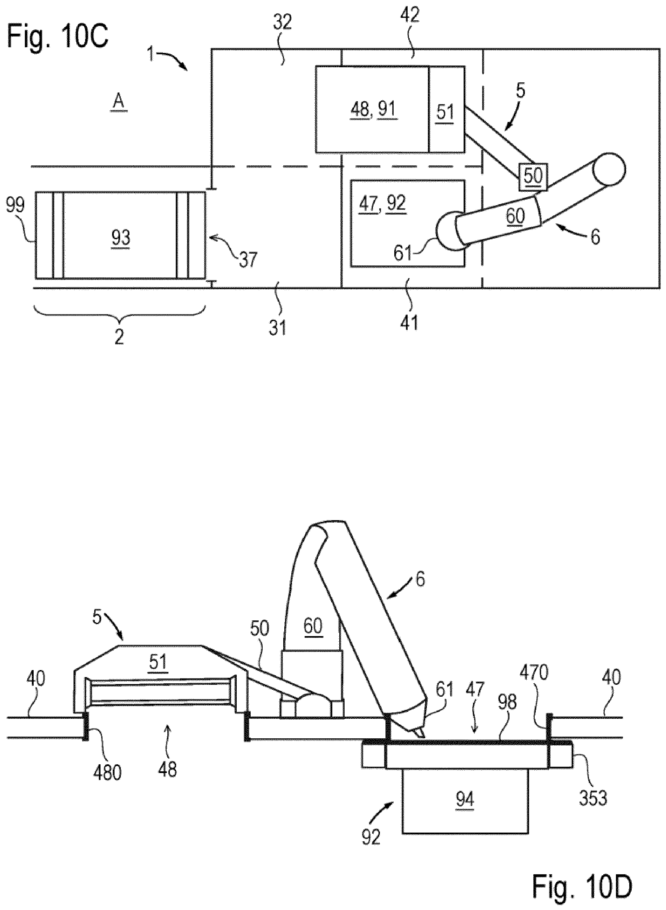

Figuren 10A bis 10D - Prozessphase 8

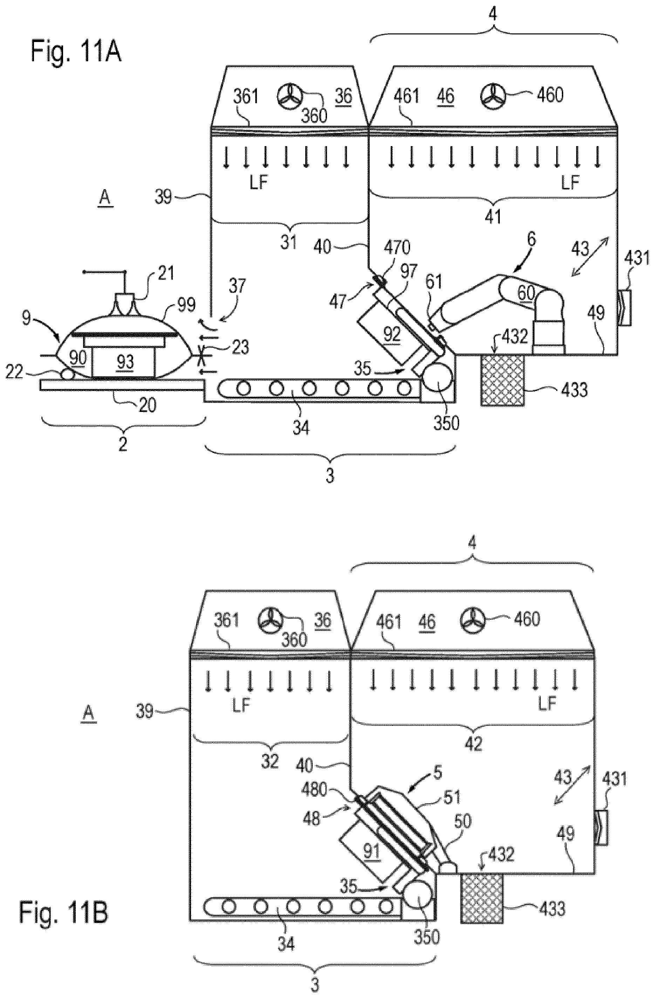

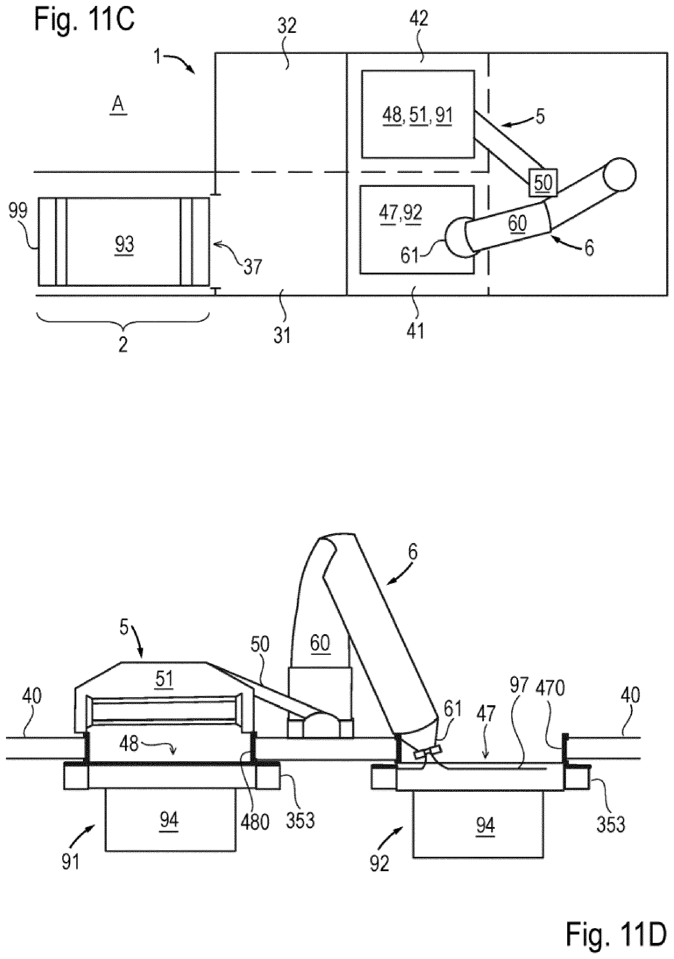

Figuren 11A bis 11D - Prozessphase 9

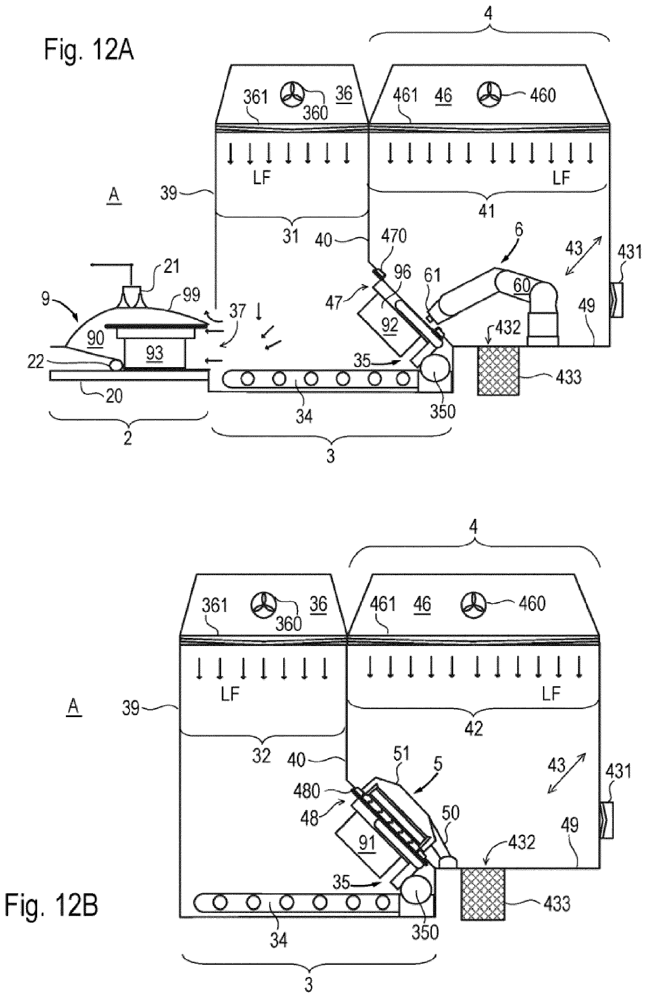

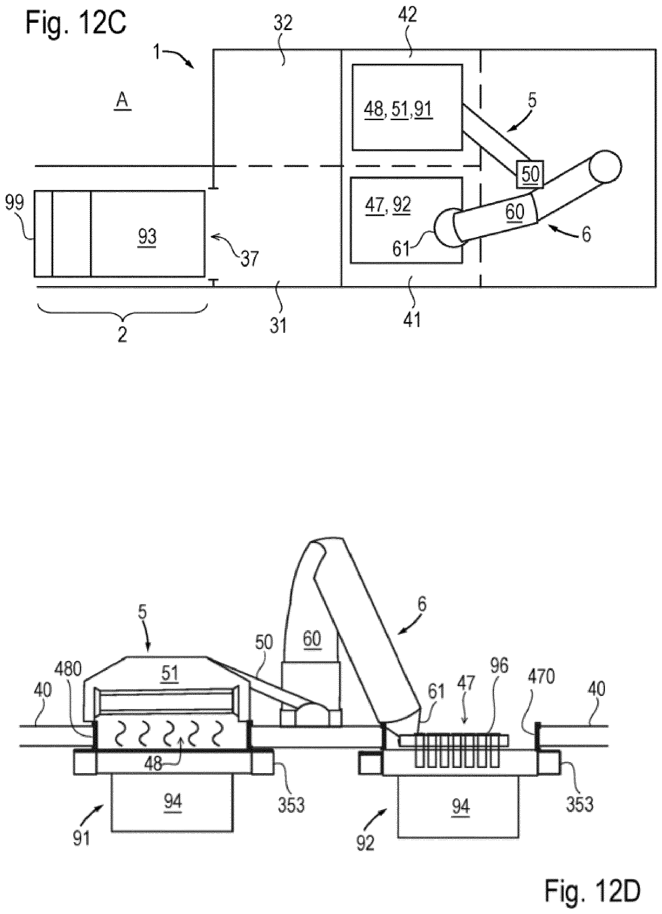

Figuren 12A bis 12D - Prozessphase 10

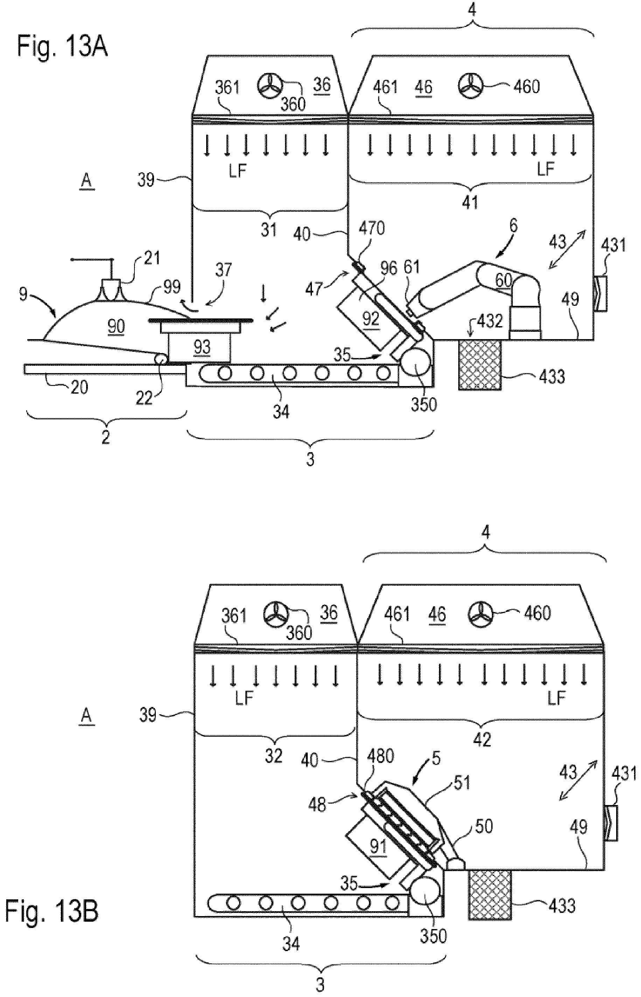

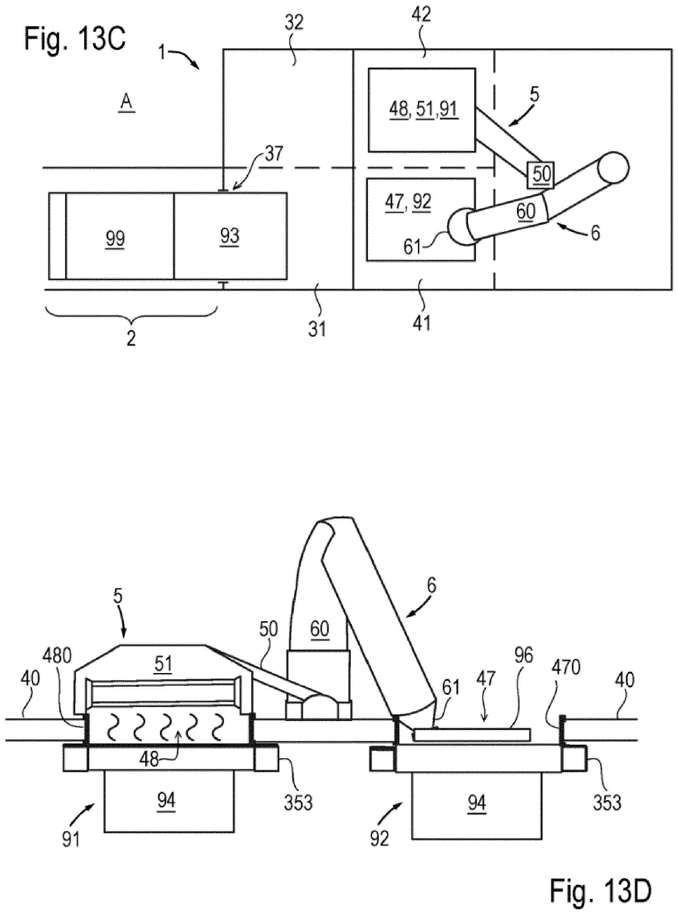

Figuren 13A bis 13D - Prozessphase 11

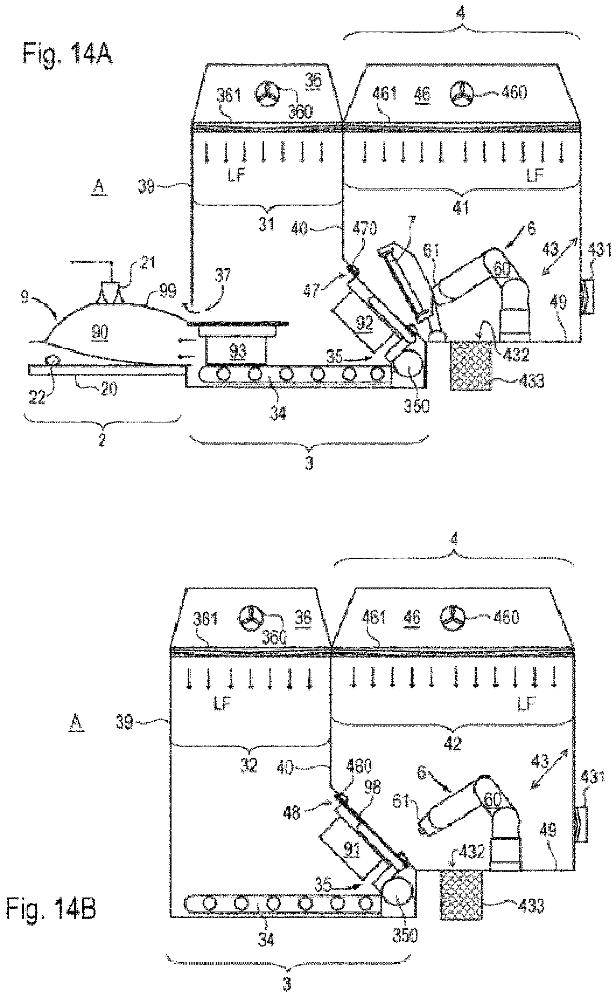

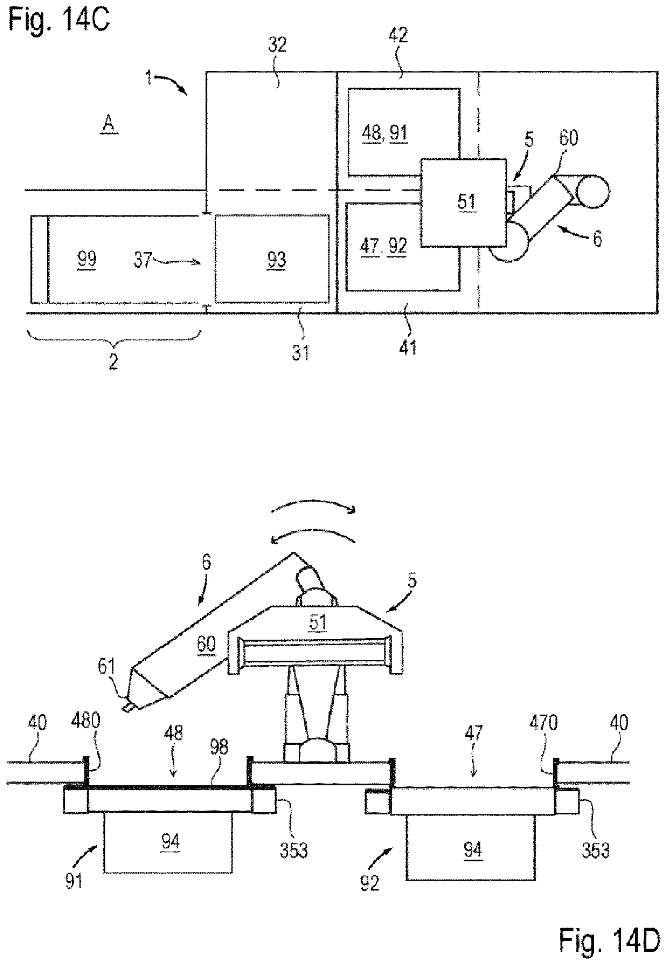

Figuren 14A bis 14D - Prozessphase 12

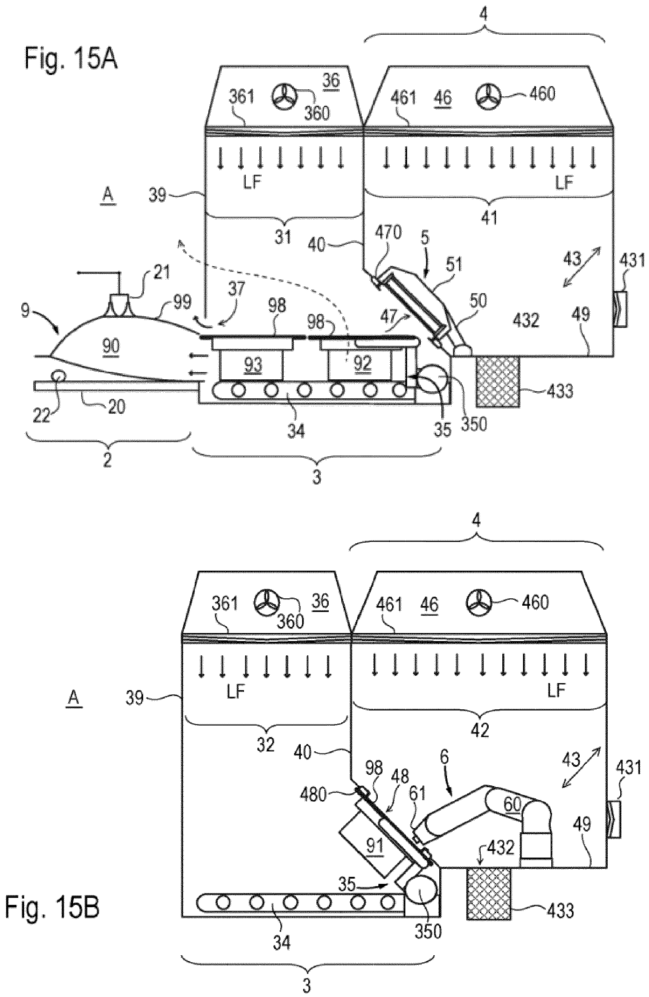

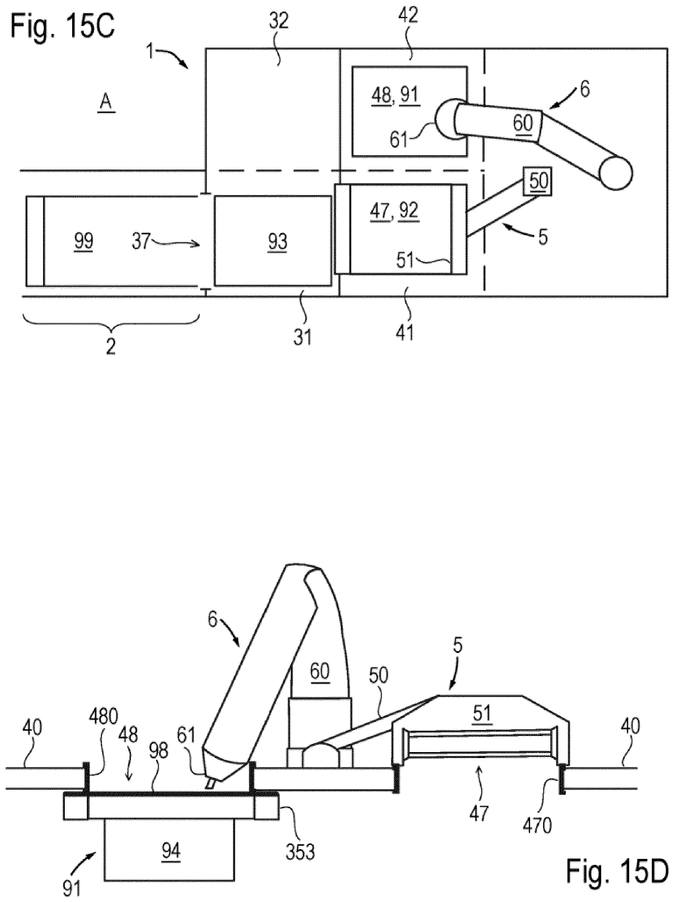

Figuren 15A bis 15D - Prozessphase 13

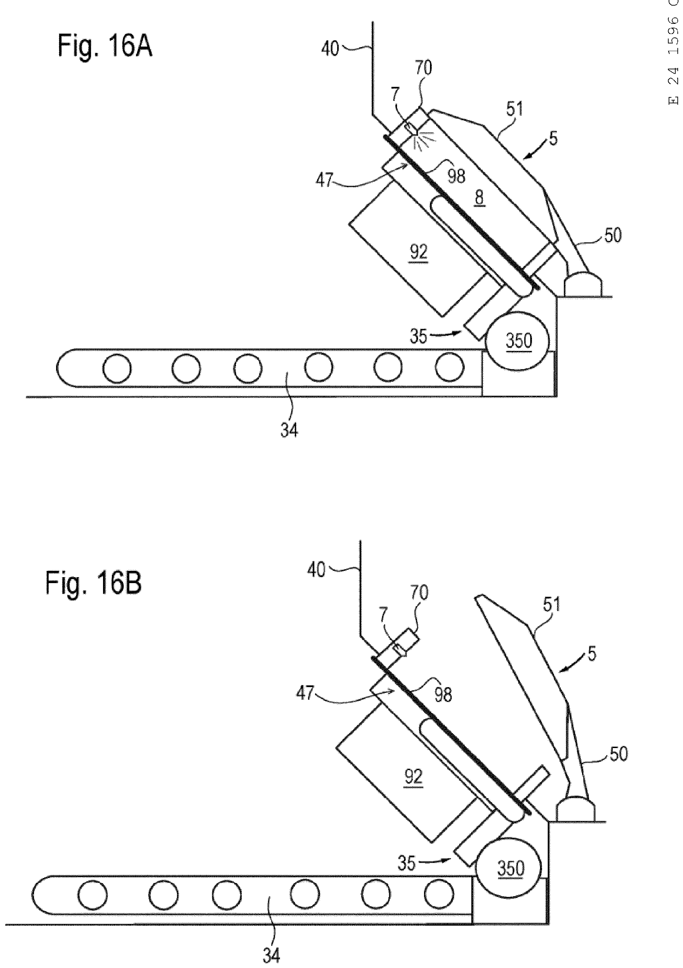

Figur 16A - eine alternative Anordnung mit der Dekantaminationsvorrichtung ausserhalb des Deckels, in einer Halterung sitzend, Gebinde an der Passage mittels der hochgeschwenkten zweiten Fördereinrichtung angedrückt, Deckel geschlossen, Dekantaminationsvorrichtung aktiviert; und
Figur 16B - die Anordnung gemäss Figur 16A mit geöffnetem Deckel und inaktiver Dekantaminationsvorrichtung.

### Ausführungsbeispiel

Mit Bezug auf die beiliegenden Zeichnungen erfolgt nachstehend die detaillierte Beschreibung des erfindungsgemässen Verfahrens und der erfindungsgemässen Schleusenanordnung zum Einschleusen von Artikeln aus einem Gebinde in die Prozesskammer eines Containments unter aseptischen Bedingungen. Wie die beigefügten Zeichnungen, betrifft die nachfolgend beschriebene Ausführungsform den Transfer von Artikeln aus Gebinden in die Prozesskammer durch zwei von der Eingangsstation in die Prozesskammer führende Passagen. Jede der Passagen lässt sich abwechselnd von einem in der Prozesskammer herangeführten Deckel verschliessen, in welchem das Emissionselement einer Dekontaminationsvorrichtung integriert sein kann.

Für die gesamte weitere Beschreibung gilt folgende Festlegung. Sind in einer Figur zum Zweck zeichnerischer Eindeutigkeit Bezugsziffern enthalten und dabei zeichnerisch eindeutig erkennbar ist, dass es sich um "wiederkehrende" Bauteile handelt, aber im unmittelbar zugehörigen Beschreibungstext nicht erläutert, so sei im Interesse der Verkürzung, auf deren Erklärung in vorangehenden Figurenbeschreibungen hingewiesen.

### Figuren 1A und 1B

Ein einzelnes Gebinde 9 umfasst:
- in Behältnis 94 mit einem aseptischen Innenraum 940 und einem Durchlass 95, der mit einer Abdeckung 98 verschlossen ist;
- ein im Innenraum 940 gelagertes Nest 96, in dessen muldenförmigen Aufnahmekonturen die Artikel 960 stecken, welche nach Öffnen der Abdeckung 98 in der Prozesskammer 43 des Containments 4 zu behandeln sind;
- optional eine zwischen der Abdeckung 98 und dem Nest 96, über den Artikeln 960 eingefügte Zwischenlage 97; sowie
- eine im Anlieferungszustand das gesamte Gebinde 9 umgebende, geschlossene Umhüllung 99, die das Innenvolumen 90 steril hält.

Das Behältnis 94 ist z.B. ein wannenförmiges Tub. Die Artikel 960 sind insbesondere Phiolen, Vials oder Spritzen. Von der Abdeckung 98 und der Umhüllung 99 bestehen vorzugsweise zumindest Flächenanteile aus einem semipermeablen Vliesgewebe, wie Tyvek^{®}. Die Abdeckung 98 ist typischerweise auf dem den Durchlass 95 des Behältnisses 94 umlaufenden Rand 941 versiegelt und bewahrt so den Innenraum 940 des Behältnisses 94 in sterilem Zustand.

### Figuren 2A und 2B

Als Bestandteil der Schleusenanordnung 1 sind die frontseitige erste Zone 31 und die rückseitige zweite Zone 32 der Eingangsstation 3 jeweils mit einer zweiten Fördereinrichtung 35 ausgestattet. Eine solche Fördereinrichtung 35 besteht aus einer Achse 350 und daran fixierten zwei kardanischen Lagern 351. Durch die Achse 350 erstreckt sich eine erste Drehachse D1. Zwei nebeneinander an der Achse 350 fixierte Gabeln 352 sind jeweils um eine zweite Drehachse D2 schwenkbar, welche sich rechtwinklig zur ersten Drehachse D1 erstrecken. In jeder Gabel 352 ist ein um eine dritte Drehachse D3 schwenkbarer Träger 353 aufgehängt, wobei die erste Drehachse D1 und die dritte Drehachse D3 zueinander parallel verlaufen. Die Träger 353 sind zum Unterfassen der Ränder 941 der Behältnisse 94 beim An- bzw. Abschwenken von den von der Eingangsstation 3 in die Prozesskammer 43 führenden Passagen 47,48 konfiguriert.

### Figuren 3A bis 3D

### Aufbau der Schleusenanordnung 1

Die im Aufstellraum A installierte Schleusenanordnung 1 strukturiert sich in eine Eingangsstation 3, eine dieser vorgelagerte Bereitstellungsstation 2 und das sich an die Eingangsstation 3 anschliessende Containment 4 mit dessen Prozesskammer 43. Die Bereitstellungsstation 2 besitzt die Auflage 20, um darauf neue, dritte Gebinde 93 mit zunächst noch mit verschlossener Umhüllung 99 zu positionieren. Das Vorschubmittel 22 dient dem Ausschieben solcher dritten Gebinde 93 aus der geöffneten Umhüllung 99 von der Auflage 20 durch den Zugang 37 im die Eingangsstation 3 umgebenden Gehäuse 39 auf die erste Fördereinrichtung 34 in der frontseitigen ersten Zone 31 der Eingangsstation 3. Während des Ausschiebens hält der in der Bereitstellungsstation 2 vorhandene Greifer 21 die Umhüllung 99 zurück. An die erste Fördereinrichtung 34 schliesst sich die zweite Fördereinrichtung 35 an. Im Plenum 36 der frontseitigen ersten Zone 31 der Eingangsstation 3 sind ein Zuluftventilator 360 sowie Zuluftfilter 361 zur Erzeugung eines gereinigten, abwärts laminar strömenden Luftstromes LF angeordnet.

Zwischen der frontseitigen ersten Zone 31 der Eingangsstation 3 und der frontseitigen ersten Zone 41 der Prozesskammer 43 erstreckt sich die Trennwand 40, welche die erste Passage 47 zwischen den Zonen 31,41 aufweist. In der ersten Passage 47 sitzt die Dichtung 470. Auch im Plenum 46 der frontseitigen ersten Zone 41 des Containments 4 sitzen ein Zuluftventilator 460 und ein Zuluftfilter 461 zur Erzeugung eines gereinigten, abwärts laminar strömenden Luftstromes LF. Im die Prozesskammer 43 umgebenden Gehäuse 49 sind seitlich und/oder alternativ in dessen Boden ein Abluftfilter 431 und im Boden eine Abfallöffnung 432 vorgesehen, die zu einem Depot 433 führt.

In der sich über die frontseitige erste Zone 41 und die rückseitige zweite Zone 42 erstreckende gemeinsame Prozesskammer 43 ist eine Apparatur 5 mit dem Ausleger 50 und dem daran gehaltenen Deckel 51 stationiert, in dem die Dekontaminationsvorrichtung 7 integriert ist. Durch Schwenken des Auslegers 50 kann der Deckel 51 über Zwischenstellungen an die erste Passage 47 oder die zweite Passage 48 herangeführt werden.

In der Eingangsstation 3 liegt hinter der ersten Zone 31 die rückseitige zweite Zone 32, und im Containment 4 liegt hinter der ersten Zone 41 die rückseitige zweite Zone 42. Äquivalent zur ersten Zone 31 ist die zweite Zone 32 ebenfalls in deren Plenum 36 mit einem Zuluftventilator 360 und einem Zuluftfilter 361 zur Erzeugung eines Luftstromes LF ausgestattet. Wiederum vorhanden sind eine erste Fördereinrichtung 34 und die sich daran anschliessende zweite Fördereinrichtung 35 an. Gleichfalls äquivalent zur ersten Zone 41 hat die zweite Zone 42 das Plenum 46 mit Zuluftventilator 460 und Zuluftfilter 461 zur Erzeugung des Luftstromes LF.

Bei kleineren Anlagen können ein gemeinsamer Zuluftventilator 360 und ein gemeinsamer Zuluftfilter 361 für die erste Zone 31 und die zweite Zone 32 sowie ein gemeinsamer Zuluftventilator 460 und ein gemeinsamer Zuluftfilter 461 für die erste Zone 41 und die zweite Zone 42 genügen. Bei grösseren Anlagen wird man für die Zonen 31,32 bzw. 41,42 mehrere Zuluftventilatoren 360,460 bzw. Zuluftfilter 361,461 vorsehen. In der rückseitigen zweiten Zone 42 sind ebenfalls im Gehäuse 49 seitlich und/oder alternativ in dessen Boden der Abluftfilter 431 und im Boden die zu einem Depot 433 führende Abfallöffnung 432 vorhanden.

Zwischen der rückseitigen zweiten Zone 32 der Eingangsstation 3 und der rückseitigen zweiten Zone 42 der Prozesskammer 43 erstreckt sich erneut die Trennwand 40, welche nun die zweite Passage 48 zwischen den Zonen 32,42 aufweist. In dieser zweiten Passage 48 sitzt die Dichtung 480. In der für die erste und zweite Zone 41,42 gemeinsamen Prozesskammer 43 ist ein Manipulator 6 mit dem Ausleger 60 und dem daran befindlichen Werkzeugkopf 61 stationiert. Durch Schwenken des Auslegers 60 kann der Werkzeugkopf 61 über Zwischenstellungen an die erste Passage 47 oder die zweite Passage 48 herangeführt werden.

### Ablauf in Prozessphase 1 (Startposition)

- In Bereitstellungsstation 2:
- Ein neues, drittes Gebinde 93, noch mit verschlossener Umhüllung 99, ist auf der Auflage 20 angeliefert, befindet sich vor dem Zugang 37 und wird von durch diesen ausfliessender Strömung LF angeströmt.
- In Eingangsstation 3, in deren frontseitiger erster Zone 31: Ein zweites Gebinde 92, bereits aus der Umhüllung 99 befreit, befindet sich auf der ersten Fördereinrichtung 34. Vonseiten der ersten Zone 31 ist die erste Passage 47 quasi offen.
- In Eingangsstation 3, rückseitige zweite Zone 32: Die zweite Passage 48 in die Prozesskammer 43 des Containments 4 ist vom angedrückten ersten Gebinde 91 mit dessen Abdeckung 98 verschlossen, jedoch bereits dekontaminiert.
- Im Containment 4, frontseitige erste Zone 41: Die erste Passage 47 in die Prozesskammer 43 ist vom Deckel 51 mit der darin integrierten Dekontaminationsvorrichtung 7 verschlossen. Der Deckel 51 wurde von der Apparatur 5 mit dem geschwenkten Ausleger 50 aus der Prozesskammer 43 heraus herangeführt.
- Im Containment 4, rückseitige zweite Zone 42: Mit einem Werkzeug, welches am Werkzeugkopf 61 eines geschwenkten Auslegers 60 des in der Prozesskammer 43 stationierten Manipulators 6 montiert ist, wird die Abdeckung 98 geöffnet, z.B. aufgeschnitten, und entfernt.

Bei der nachstehenden Beschreibung des Ablaufs in den weiteren Prozessphasen 2 bis 13 (Figuren 4A bis 15D) werden zur Vermeidung von Wiederholungen jeweils nur die Veränderungen zur vorherigen Prozessphase aufgelistet.

### Ablauf in Prozessphase 2 (Figuren 4A bis 4D)

- In Eingangsstation 3, frontseitige erste Zone 31: Das zweite Gebinde 92, ohne Umhüllung 99, noch auf der ersten Fördereinrichtung 34 stehend, wird von der zweiten Fördereinrichtung 35 übernommen.

### Ablauf in Prozessphase 3 (Figuren 5A bis 5D)

- In Bereitstellungsstation 2: Die bis anhin verschlossene Umhüllung 99 um das neue, dritte Gebinde 93 wird mittels des Werkzeugs 23 geöffnet, z.B. aufgeschnitten; hierbei hält der Greifer 21 die Umhüllung 99. Alternativ kann ein zuvor an der Umhüllung 99 angebrachter Schnitt, den man zunächst, z.B. zuklemmt, jetzt freigegeben werden.
- In Eingangsstation 3, frontseitige erste Zone 31: Von der zweiten Fördereinrichtung 35 wurde das zweite Gebinde 92 an die zweite Passage 48 herangeführt, welche nun beidseitig vom zweiten angedrückten Gebinde 92 bzw. aufgesetzten Deckel 51 verschlossen ist.
- Im Containment 4, rückseitige zweite Zone 42: Mit einem Werkzeug, das am Werkzeugkopf 61 des schwenkbaren Auslegers 60 des Manipulators 6 installiert ist, wird bei offener Abdeckung 98 die optional vorhandene Zwischenlage 97 aus dem ersten Gebinde 91 entnommen.

### Ablauf in Prozessphase 4 (Figuren 6A bis 6D)

- In Bereitstellungsstation 2: Das neue, dritte Gebinde 93 wird aus der aufseiten des Zugangs 37 offenen Umhüllung 99 vom bewegten Vorschubmittel 22 sukzessive in Richtung Zugang 37 ausgeschoben und dabei von der gereinigten Strömung LF bestrahlt. Dies verhindert das Umspülen des Gebindes 93 mit viel unreiner Umgebungsluft aus dem Aufstellraum A. Vom Greifer 21 wird die Umhüllung 99 festgehalten.
- Im Containment 4, frontseitige erste Zone 41: Die Dekontaminationsvorrichtung 7 wird aktiviert, um das zwischen Deckel 51, Abdeckung 98 auf erstem Gebinde 91, der die erste Passage 47 umrandende Dichtung 470 und möglichen freiliegenden Wandungsflächen im Umfeld 8 vorhandene Gasvolumen und alle davon umspülten Oberflächen zu dekontaminieren.
- Im Containment 4, rückseitige zweite Zone 42: Mit einem vom Manipulator 6 herangeführten Werkzeug werden aus dem Innenvolumen 90 aus dem Behältnis 94 des ersten Gebindes 91 das Nest 96, zusammen mit den darin gelagerten Artikeln 960 oder nur die Artikel 960, in die Prozesskammer 43 zur weiteren Verarbeitung transferiert.

### Ablauf in Prozessphase 5 (Figuren 7A bis 7D)

- In Bereitstellungsstation 2 und Eingangsstation 3, frontseitige erste Zone 31: Das neue, dritte Gebinde 93 wird durch pushende Bewegung des Vorschubmittels 22 aus der Umhüllung 99 heraus, durch den Zugang 37 im Gehäuse 39 und gegen die gereinigte Strömung LF, auf die erste Fördereinrichtung 34 in die erste Zone 31 der Eingangsstation 3 geschoben. Das Ausschieben des dritten Gebindes 93 aus der Umhüllung 99 geschieht direkt auf die erste Fördereinrichtung 34, um eine Berührung zwischen Gebinde 93 und Auflage 20 zu vermeiden und so keine Übertragung von Verunreinigungen zu erlauben. Der Greifer 21 hält dabei die Umhüllung 99 zwecks Entsorgung zurück.
- Im Containment 4, rückseitige zweite Zone 42: In das in vorheriger Prozessphase geleerte Behältnis 94 wird ein aus früherem oder gegenwärtigem Produktionszyklus aus der Prozesskammer 43 stammendes Nest 96 mittels des Manipulators 6 in dieses Behältnis 94 rückgeführt.

### Ablauf in Prozessphase 6 (Figuren 8A bis 8D)

- In Bereitstellungsstation 2 und Eingangsstation 3, frontseitige erste Zone 31: Das neue, dritte Gebinde 93 ist komplett in die erste Zone 31 eingeschoben und wird von erster Fördereinrichtung 34 getragen. Auf der Auflage 20 verbleiben die vom Greifer 21 zurückgehaltene und zur Entsorgung bestimmte Umhüllung 99 sowie das in Ausgangsposition zurückbewegte Vorschubmittel 22.
- Im Containment 4, frontseitige erste Zone 41: Die im Deckel 51 integrierte Dekontaminationsvorrichtung 7 kann abgeschaltet werden; die Behandlung ist abgeschlossen. Mit Abschwenken des Auslegers 50 der Apparatur 5 wird der Deckel 51 von der ersten Passage 47 abgehoben. Zugleich wird der Werkzeugkopf 61 des Manipulators 6 hin zur ersten Passage 47 bewegt.
- Im Containment 4, rückseitige zweite Zone 42: Mit Umschwenken des Auslegers 50 der Apparatur 5 wird der Deckel 51 der zweiten Passage 48 angenähert.

### Ablauf in Prozessphase 7 (Figuren 9A bis 9D)

- Im Containment 4, frontseitige erste Zone 41: Der Deckel 51 ist von der ersten Passage 47 entfernt und das vom Manipulator 6 geführte Werkzeug wird der Abdeckung 98 des zweiten Gebindes 92 angenähert.
- Im Containment 4, rückseitige zweite Zone 42 und Eingangsstation 3, rückseitige zweite Zone 32: Mit weiterem Schwenken des Auslegers 50 der Apparatur 5 gelangt der Deckel 51 mit der darin integrierten Dekontaminationsvorrichtung 7 auf die zweite Passage 48. Anschliessend wird mit Abschwenken der zweiten Fördereinrichtung 35 das Behältnis 94 des verarbeiteten ersten Gebindes 91 von der zweiten Passage 48 entfernt und auf die erste Fördereinrichtung 34 zum Abtransport nach ausserhalb der Schleusenanordnung 1 aufgesetzt.

### Ablauf in Prozessphase 8 (Figuren 10A bis 10D)

- In Bereitstellungsstation 2: Wie in Prozessphase 1 gemäss Startposition wird ein nächstes neues, drittes Gebinde 93, wiederum mit noch verschlossener Umhüllung 99 auf der Auflage 20, vor dem Zugang 37, positioniert.
- Im Containment 4, frontseitige erste Zone 41: Mit dem am Werkzeugkopf 61 des Manipulators 6 installierten Werkzeug wird die Abdeckung 98 des zweiten Gebindes 92, welches an der ersten Passage 47 angedrückt ist, z.B. durch Aufschneiden geöffnet und der dabei entstehende Schnittabfall entfernt, so dass Zugang zum Durchlass 95 in das Behältnis 94 geschaffen ist.
- In Eingangsstation 3, rückseitige zweite Zone 32: Ein auf der Bereitstellungsstation 2 von der Umhüllung 99 befreites, weiteres erstes Gebinde 91 mit intakter Abdeckung 98 wurde quer auf die erste Fördereinrichtung 34 transferiert und inzwischen auch auf die zweite Fördereinrichtung 35 geladen.

### Ablauf in Prozessphase 9 (Figuren 11A bis 11D)

- In Bereitstellungsstation 2: Wie in Prozessphase 3 gemäss Figur 5A wird mittels des Werkzeugs 23 die bis anhin verschlossene Umhüllung 99 geöffnet.
- Im Containment 4, frontseitige erste Zone 41: Wie in Prozessphase 3 gemäss Figur 5B wird mit dem vom Manipulator 6 geführten Werkzeug, bei offener Abdeckung 98, die optional vorhandene Zwischenlage 97 aus dem zweiten Gebinde 92, das an der ersten Passage 47 angedrückt ist, entnommen.
- In Eingangsstation 3, rückseitige zweite Zone 32: Durch Aufschwenken der zweiten Fördereinrichtung 35 wird das weitere erste, quer transferierte Gebinde 91 mit der Abdeckung 98 an die zweite Passage 48 angedrückt. Somit wird wieder, wie bereits in Prozessphase 3 gemäss Figur 5A, an der ersten Passage 47 entstanden, jetzt an der zweiten Passage 48, ein zwischen Deckel 51, Abdeckung 98 des zweiten Gebindes 92, der die zweite Passage 48 umrandenden Dichtung 480 und möglichen freiliegenden Wandungsflächen ein Umfeld 8 samt davon eingeschlossenes und in der nächsten Prozessphase 10 zu dekontaminierendes Gasvolumen gebildet.

### Ablauf in Prozessphase 10 (Figuren 12A bis 12D)

- In Bereitstellungsstation 2: Hier wiederholt sich die Situation von Prozessphase 4 gemäss Figur 6A, allerdings nun mit dem nächsten neuen, dritten Gebinde 93.
- Im Containment 4, frontseitige erste Zone 41: Hier wiederholt sich die Situation von Prozessphase 4 gemäss Figur 6B, allerdings nun mit dem zweiten Gebinde 92, welches jetzt an der ersten Passage 47 angedrückt ist.
- In Eingangsstation 3, rückseitige zweite Zone 32, und Containment 4, rückseitige zweite Zone 42: Das in der vorherigen Prozessphase 9 gemäss Figur 11B gebildete Umfeld 8 mit darin eingeschlossenem Gasvolumen wird mittels der aktivierten Dekontaminationsvorrichtung 7 dekontaminiert.

### Ablauf in Prozessphase 11 (Figuren 13A bis 13D)

- In Bereitstellungsstation 2: Hier wiederholt sich die Situation von Prozessphase 5 gemäss Figur 7A, allerdings nun mit dem nächsten neuen, dritten Gebinde 93.
- Im Containment 4, frontseitige erste Zone 41: Hier wiederholt sich die Situation von Prozessphase 5 gemäss Figur 7B, allerdings wird nun in das Behältnis 94 des zweiten Gebindes 92, welches jetzt an der ersten Passage 47 angedrückt ist, ein leeres Nest 96 aus der Prozesskammer 43 zurückgeführt.
- In Eingangsstation 3, rückseitige zweite Zone 32, und Containment 4, rückseitige zweite Zone 42: Die Dekontamination läuft weiter.

### Ablauf in Prozessphase 12 (Figuren 14A bis 14D)

- In Bereitstellungsstation 2: Hier wiederholt sich die Situation von Prozessphase 6 gemäss Figur 8A, allerdings nun mit dem nächsten neuen, dritten Gebinde 93.
- Im Containment 4, frontseitige erste Zone 41: Hier wiederholt sich die Situation von Prozessphase 6 gemäss Figur 8B, allerdings nun mit dem zweiten Gebinde 92 und an der ersten Passage 47, der mit Umschwenken des Auslegers 50 der Apparatur 5 der Deckel 51 angenähert wird.
- In Eingangsstation 3, rückseitige zweite Zone 32, und Containment 4, rückseitige zweite Zone 42: Die Dekontamination im Umfeld 8 an der zweiten Passage 48 mit dem weiteren ersten, quer transferierten Gebinde 91 ist beendet. Der Deckel 51 mit der Dekontaminationsvorrichtung 7 sind weggeschwenkt und der Manipulator 6 mit den am Werkzeugkopf 61 installierten Werkzeugen ist an die zweite Passage 48 herangeschwenkt.

### Ablauf in Prozessphase 13 (Figuren 15A bis 15D)

- In Eingangsstation 3, frontseitige erste Zone 31 und Containment 4, frontseitige erste Zone 41: Zuerst schwenkt die Apparatur 5 den Deckel 51 auf die erste Passage 47, diese verschliessend. Anschliessend wird durch Abschwenken der zweiten Fördereinrichtung 35 das Behältnis 94 des verarbeiteten zweiten Gebindes 92 mit dem rückgeführten Nest 96 auf die erste Fördereinrichtung 34 zwecks querverlaufendem Abtransport nach ausserhalb der Schleusenanordnung 1 geladen. Somit wird eine Kollision mit dem nächsten neuen, dritten Gebinde 93 vermieden, welches bereits auf der ersten Fördereinrichtung 34 steht.
- In Eingangsstation 3, rückseitige zweite Zone 32 und Containment 4, rückseitige zweite Zone 42: Hier wiederholt sich die Situation wie in Prozessphase 1 gemäss Figur 3B, mit einem Werkzeug am Manipulator 6 wird die Abdeckung 98 geöffnet, allerdings jetzt des ursprünglich quer auf die erste Fördereinrichtung 34 transferierten neuen ersten Gebindes 91.

### Figuren 16A und 16B

Bei dieser alternativen Anordnung ist die Dekontaminationsvorrichtung 7 nicht, wie bis anhin, im Deckel 51 integriert, sondern das Emissionselement der Dekontaminationsvorrichtung 7, z.B. eine Vernebelungsdüse zum Eintrag eines H2 O2 -Aerosols, sitzt nicht im Deckel 51, sondern ausserhalb in einer Halterung 70. Diese Halterung 70 kann in einfachster Gestalt von einer Abkantung der zwischen Eingangsstation 3 Containment 4 sich erstreckenden Trennwand 40 gebildet sein. Die dem Umfeld 8 zugewandte Oberfläche der Halterung 70 ist dann Bestandteil des im Produktionsprozess zu dekontaminierenden eingeschlossenen Gasvolumens und Umfeld 8. Unverändert wird der Deckel 51 von einem schwenkbaren Ausleger 50 einer in der Prozesskammer 43 stationierten Apparatur 5 getragen.

Bei einer ersten bevorzugten Anwendung der Erfindung kann vorgesehen sein, dass a) die Dekontaminationsvorrichtung 7 im Deckel 51 integriert oder ausserhalb des Deckels 51 angeordnet ist; und b) das Emissionselement der Dekontaminationsvorrichtung 7 direkt im Deckel 51 oder ausserhalb des Deckels 51 in einer Aufnahme 70 angeordnet ist.

Bei einer zweiten bevorzugten Anwendung der Erfindung kann alternativ oder zusätzlich zur ersten bevorzugten Anwendung vorgesehen sein, dass das eingeschlossene Umfeld 8 das darin vorhandene Gasvolumen und vom Gasvolumen umspülte Oberflächen umfasst, nämlich Oberflächen: a) des Deckels 51; b) der optionalen Aufnahme 70 ; c) einer optionalen Dichtung 470,480 ; und d) von optional freiliegenden Wandungsflächen.

Bei einer dritten bevorzugten Anwendung der Erfindung kann alternativ oder zusätzlich zur ersten bis zweiten bevorzugten Anwendung vorgesehen sein, dass a) die Prozesskammer 43 mit einem Manipulator 6 oder zusätzlich mit einem zweiten Manipulator 6 ausgestattet ist, an deren schwenkbaren Auslegern 60 jeweils ein mit Werkzeugen bestückter Werkzeugkopf 61 sitzt, wobei: b) die Werkzeuge ausgebildet sind, um die Abdeckung 98 zu öffnen und anschliessend die Artikel 960 aus dem Behältnis 94 durch den freigelegten Durchlass 95 in die Prozesskammer 43 zu transferieren; und c) der eine Manipulator 6 mit dessen Werkzeugen wechselhaft an den Passagen 47,48 zum Öffnen der Abdeckung 98 und Transfer der Artikel 960 in die Prozesskammer 43 zum Einsatz kommt; oder d) der eine Manipulator 6 und der zweite Manipulator 6 jeweils nur an einer der Passagen 47,48 oder die beiden Manipulatoren 6 wechselhaft an den Passagen 47,48 zum Einsatz kommen.

Bei einer vierten bevorzugten Anwendung der Erfindung kann alternativ oder zusätzlich zur ersten bis dritten bevorzugten Anwendung vorgesehen sein, dass a) ein Schneidwerkzeug Komponente der Werkzeuge ist, um für das Öffnen die Abdeckung 98 aufzuschneiden; wobei b) vorzugsweise von der Abdeckung 98 und der Umhüllung 99 zumindest Flächenanteile aus einem semipermeablen Vliesgewebe, wie Tyvek^{®} bestehen, und die Abdeckung 98 vor dem Entfernen den Innenraum 940 des Behältnisses 94 in sterilem Zustand bewahrt und auf einem den Durchlass 95 des Behältnisses 94 umlaufenden Rand 941 versiegelt ist.

Bei einer fünften bevorzugten Anwendung der Erfindung kann alternativ oder zusätzlich zur ersten bis vierten bevorzugten Anwendung vorgesehen sein, dass a) im Behältnis 94 zur Aufnahme einer Vielzahl von Artikeln 960 ein entnehmbares Nest 96 lagert, welches zusammen mit den Artikeln 960 in die Prozesskammer 43 transferiert wird; und b) nach Leerung des Nestes 96 von den Artikeln 960 in der Prozesskammer 43 , dieses geleerte Nest 96 aus der Prozesskammer 43 in ein nächstes oder dasselbe an der betreffenden Passage 47,48 angedrücktes geleertes Behältnis 94 entsorgt wird; oder c) die Artikel 960 direkt aus dem im Behältnis 94 verbleibenden Nest 96 in die Prozesskammer 43 transferiert werden.

Bei einer sechsten bevorzugten Anwendung der Erfindung kann alternativ oder zusätzlich zur ersten bis fünften bevorzugten Anwendung vorgesehen sein, dass die Dekontaminationsvorrichtung vorzugsweise als Strahlungsquelle, z.B. UVC, UV, E-Beam oder Puls-Licht, oder alternativ als eine Begasungseinrichtung, z.B zur Verneblung einer H 2 O 2 - Lösung, ausgebildet wird.

Bei einer siebten bevorzugten Anwendung der Erfindung kann alternativ oder zusätzlich zur ersten bis sechsten bevorzugten Anwendung vorgesehen sein, dass der Eingangsstation 3 , welche von einem Gehäuse 39 umgeben ist, das einen zu einer angrenzenden Bereitstellungsstation 2 führenden Zugang 37 aufweist, wobei: a) die Eingangsstation 3 mit einer gereinigten, gleichgerichteten Verdrängungsströmung LF betrieben wird, die teilweise durch den Zugang 37 austritt; b) an der Bereitstellungsstation 2 die Gebinde 9;91,92,93 angeliefert werden, welche im Anlieferungszustand jeweils von einer Umhüllung 99 umgeben sind, um das Innenvolumen 90 des Gebindes 9;91,92,93 mit dessen Inhalt aseptisch zu erhalten; und c) an der Bereitstellungsstation 2 eine an der Umhüllung 99 angebrachte Öffnung, die man bis anhin geschlossen hält, nun auf der der aus dem Zugang 37 austretenden Verdrängungsströmung LF zugewandten Seite freigegeben wird, um das einzelne Gebinde 9;91,92,93 in die Eingangsstation 3 einzubringen; oder d) an der Bereitstellungsstation 2 die Umhüllung 99 auf der der aus dem Zugang 37 austretenden Verdrängungsströmung LF zugewandten Seite aufgetrennt wird, um das einzelne Gebinde 9;91,92,93 in die Eingangsstation 3 einzubringen.

Bei einer achten bevorzugten Anwendung der Erfindung kann zusätzlich zur siebten bevorzugten Anwendung vorgesehen sein, dass a) die Positionierung des in der zuerst noch geschlossenen Umhüllung 99 steckenden Gebindes 9;91,92,93 auf einer Auflage 20 erfolgt; b) zum Ausschieben des Gebindes 9;91,92,93 aus der offenen Umhüllung 99 , bei auf das Gebinde 9;91,92,93 fliessender Verdrängungsströmung LF , ein Vorschubmittel 22 dient; und c) die Umhüllung 99 von einem Greifer 21 , vorzugsweise als Sauger ausgebildet, gehalten wird.

Bei einer neunten bevorzugten Anwendung der Erfindung kann alternativ oder zusätzlich zur ersten bis achten bevorzugten Anwendung vorgesehen sein, dass a) die in die Eingangsstation 3 eingebrachten Gebinde 9;91,92,93 mittels zumindest einer Fördereinrichtung 34,35 den Passagen 47,48 zugeführt werden; und b) die zumindest eine Fördereinrichtung 34,35 vorzugsweise auf dem Prinzip der Magnetschwebetechnik beruht. Bei einer zehnten bevorzugten Anwendung der Erfindung kann zusätzlich zur neunten bevorzugten Anwendung vorgesehen sein, dass a) mittels einer ersten Fördereinrichtung 34 der Transport der Gebinde 9;91,92,93 innerhalb der Eingangsstation 3 erfolgt; während b) eine zweite Fördereinrichtung 35 das Heranschwenken der Gebinde 9;91,92,93 mit an der jeweiligen Passage 47,48 angepressten Abdeckung 98 bewirkt; und c) die zweite Fördereinrichtung 35 auch zum Entfernen bearbeiteter Gebinde 9;91,92,93 von der jeweiligen Passage 47,48 nutzbar ist.

Bei einer elften bevorzugten Anwendung der Erfindung kann zusätzlich zur zehnten bevorzugten Anwendung vorgesehen sein, dass zur Positionierung der Gebinde 9;91,92,93 an der jeweiligen Passage 47,48 die zweite Fördereinrichtung 35 ein kardanisches Lager 351 umfasst.

Bei einer zwölften bevorzugten Anwendung der Erfindung kann alternativ oder zusätzlich zur zehnten oder elften bevorzugten Anwendung vorgesehen sein, dass die zweite Fördereinrichtung 35 besteht aus: a) einer Achse 350 , durch welche sich eine erste Drehachse D1 erstreckt; b) zwei nebeneinander an der Achse 350 fixierten Gabeln 352 , die jeweils um eine zweite Drehachse D2 schwenkbar sind, welche sich rechtwinklig zur ersten Drehachse D1 erstrecken; und c) jeweils einen in der zugehörigen Gabel 352 aufgehängten und um eine dritte Drehachse D3 schwenkbaren Träger 353 , wobei die erste Drehachse D1 und die dritte Drehachse D3 zueinander parallel verlaufen und die Träger 353 zum Unterfassen der Ränder 941 der Behältnisse 94 beim An- bzw. Abschwenken von den Passagen 47,48 konfiguriert sind; wobei d) zwei nebeneinander liegende kardanische Lager 351 jeweils gebildet sind aus einen U-förmigen Träger 353 , der in der dritten Drehachse D3 jeweils mit einer Gabel 352 verbunden ist, die in der zweiten Drehachse D2 aufgehängt ist.

Bei einer dreizehnten bevorzugten Anwendung der Erfindung kann alternativ oder zusätzlich zur ersten bis zwölften bevorzugten Anwendung vorgesehen sein, dass in jeder Passage 47,48 eine Dichtung 470,480 sitzt, welche zusammen mit der Abdeckung 98 eines temporär angepressten Gebindes 9;91,92,93 und/oder mit einem temporär angepressten Deckel 51 an den Passagen 47,48 , im Übergang von der Eingangsstation 3 zur Prozesskammer 43 , Abdichtungen schafft.

Bei einer vierzehnten bevorzugten Anwendung der Erfindung kann alternativ oder zusätzlich zur ersten bis dreizehnten bevorzugten Anwendung vorgesehen sein, dass der Deckel 51 von einem schwenkbaren Ausleger 50 einer in der Prozesskammer 43 positionierten Apparatur 5 getragen wird.

Bei einer fünfzehnten bevorzugten Anwendung der Erfindung kann alternativ oder zusätzlich zu den bevorzugten Anwendungen drei bis vierzehn vorgesehen sein, dass der zumindest eine Manipulator 6 oder auch der zusätzliche Manipulator 6 als Roboter ausgebildet sind.

Bei einer sechzehnten bevorzugten Anwendung der Erfindung kann alternativ oder zusätzlich zur ersten bis fünfzehnten bevorzugten Anwendung vorgesehen sein, dass die Behältnisse 94 der Gebinde 9;91,92,93 die Gestalt wannenförmiger Tubs haben und die Artikel 960, z.B. Phiolen, Vials oder Spritzen sind, die in systematisch angeordneten Aufnahmekonturen von Nestern 96 stecken.

Bei einer ersten bevorzugten Ausgestaltung der Schleusenanordnung kann alternativ oder zusätzlich vorgesehen sein, dass a) die Dekontaminationsvorrichtung 7 im Deckel 51 integriert oder ausserhalb des Deckels 51 angeordnet ist; und b) das Emissionselement der Dekontaminationsvorrichtung 7 direkt im Deckel 51 oder ausserhalb des Deckels 51 in einer Aufnahme 70 angeordnet ist.

Bei einer zweiten bevorzugten Ausgestaltung der Schleusenanordnung kann alternativ oder zusätzlich zur ersten bevorzugten Ausgestaltung vorgesehen sein, dass das eingeschlossene Umfeld 8 das darin vorhandene Gasvolumen und vom Gasvolumen umspülte Oberflächen umfasst, nämlich Oberflächen: a) des Deckels 51; b) der optionalen Aufnahme 70 ; c) einer optionalen Dichtung 470,480 ; und d) von optional freiliegenden Wandungsflächen.

Bei einer dritten bevorzugten Ausgestaltung der Schleusenanordnung kann alternativ oder zusätzlich zur ersten oder zweiten bevorzugten Ausgestaltung vorgesehen sein, dass a) die Prozesskammer 43 mit einem Manipulator 6 oder zusätzlich mit einem zweiten Manipulator 6 ausgestattet ist, an deren schwenkbaren Auslegern 60 jeweils ein mit Werkzeugen bestückter Werkzeugkopf 61 sitzt, wobei: b) die Werkzeuge ausgebildet sind, um die Abdeckung 98 zu öffnen und anschliessend die Artikel 960 aus dem Behältnis 94 durch den freigelegten Durchlass 95 in die Prozesskammer 43 zu transferieren; und c) der eine Manipulator 6 mit dessen Werkzeugen wechselhaft an den Passagen 47,48 zum Öffnen der Abdeckung 98 und Transfer der Artikel 960 in die Prozesskammer 43 zum Einsatz kommt; oder d) der eine Manipulator 6 und der zweite Manipulator 6 jeweils nur an einer der Passagen 47,48 oder die beiden Manipulatoren 6 wechselhaft an den Passagen 47,48 zum Einsatz kommen.

Bei einer vierten bevorzugten Ausgestaltung der Schleusenanordnung kann alternativ oder zusätzlich zur ersten bis dritten bevorzugten Ausgestaltung vorgesehen sein, dass a) die Werkzeuge ein Schneidwerkzeug als Komponente aufweisen, bestimmt zum Öffnen der Abdeckung 98 ; wobei b) vorzugsweise von der Abdeckung 98 und der Umhüllung 99 zumindest Flächenanteile aus einem semipermeablen Vliesgewebe, wie Tyvek^{®} bestehen, und die Abdeckung 98 vor dem Entfernen den Innenraum 940 des Behältnisses 94 in sterilem Zustand bewahrt und auf einem den Durchlass 95 des Behältnisses 94 umlaufenden Rand 941 versiegelt ist.

Bei einer fünften bevorzugten Ausgestaltung der Schleusenanordnung kann alternativ oder zusätzlich zur ersten bis vierten bevorzugten Ausgestaltung vorgesehen sein, dass a) im Behältnis 94 zur Aufnahme einer Vielzahl von Artikeln 960 ein entnehmbares Nest 96 lagert, welches zusammen mit den Artikeln 960 in die Prozesskammer 43 transferierbar ist; und b) das von Artikeln 960 geleerte Nest 96 aus der Prozesskammer 43 in ein nächstes oder dasselbe an der betreffenden Passage 47,48 angedocktes geleertes Behältnis 94 entsorgbar ist; oder c) die Artikel 960 direkt aus dem im Behältnis 94 verbleibenden Nest 96 in die Prozesskammer 43 transferierbar sind.

Bei einer sechsten bevorzugten Ausgestaltung der Schleusenanordnung kann alternativ oder zusätzlich zur ersten bis fünften bevorzugten Ausgestaltung vorgesehen sein, dass die Dekontaminationsvorrichtung vorzugsweise als Strahlungsquelle, z.B. UVC, UV, E-Beam oder Puls-Licht, oder alternativ als eine Begasungseinrichtung, z.B. zur Verneblung einer H 2 O 2 -Lösung, ausgebildet ist.

Bei einer siebten bevorzugten Ausgestaltung der Schleusenanordnung kann alternativ oder zusätzlich zur ersten bis sechsten bevorzugten Ausgestaltung vorgesehen sein, dass der Eingangsstation 3 , welche von einem Gehäuse 39 umgeben ist, das einen zu einer angrenzenden Bereitstellungsstation 2 führenden Zugang 37 aufweist, wobei: a) die Eingangsstation 3 mit einer gereinigten, gleichgerichteten Verdrängungsströmung LF betreibbar ist, die teilweise durch den Zugang 37 austritt; b) die Bereitstellungsstation 2 zur Anlieferung der Gebinde 9;91,92,93 bestimmt ist, welche im Anlieferungszustand jeweils von einer Umhüllung 99 umgeben sind, um das Innenvolumen 90 des Gebindes 9;91,92,93 mit dessen Inhalt aseptisch zu erhalten; und c) an der Bereitstellungsstation 2 eine an der Umhüllung 99 angebrachte Öffnung, die bis anhin mittels eines angesetzten Werkzeugs geschlossen bleibt, nun auf der der aus dem Zugang 37 austretenden Verdrängungsströmung LF zugewandten Seite durch Lösen des Werkzeugs freikommt, so dass das einzelne Gebinde 9;91,92,93 in die Eingangsstation 3 einbringbar ist; oder d) an der Bereitstellungsstation 2 die Umhüllung 99 auf der der aus dem Zugang 37 austretenden Verdrängungsströmung LF zugewandten Seite mittels eines Werkzeugs auftrennbar ist, so dass das einzelne Gebinde 9;91,92,93 in die Eingangsstation 3 einbringbar ist.

Bei einer achten bevorzugten Ausgestaltung der Schleusenanordnung kann zusätzlich zur siebten bevorzugten Ausgestaltung vorgesehen sein, dass a) zur Positionierung des in der zuerst noch geschlossenen Umhüllung 99 steckenden Gebindes 9;91,92,93 eine Auflage 20 vorgesehen ist; b) zum Ausschieben des Gebindes 9;91,92,93 aus der offenen Umhüllung 99 , bei auf das Gebinde 9;91,92,93 fliessender Verdrängungsströmung LF , ein Vorschubmittel 22 dient; und c) zum Zurückhalten der Umhüllung 99 ein Greifer 21 , vorzugsweise als Sauger ausgebildet, dient.

Bei einer neunten bevorzugten Ausgestaltung der Schleusenanordnung kann alternativ oder zusätzlich zur ersten bis achten bevorzugten Ausgestaltung vorgesehen sein, dass a) zumindest eine Fördereinrichtung 34,35 zur Zuführung der in die Eingangsstation 3 eingebrachten Gebinde 9;91,92,93 an die Passagen 47,48 vorgesehen ist; und b) die zumindest eine Fördereinrichtung 34,35 auf dem Prinzip der Magnetschwebetechnik beruht.

Bei einer zehnten bevorzugten Ausgestaltung der Schleusenanordnung kann zusätzlich zur neunten bevorzugten Ausgestaltung vorgesehen sein, dass a) zum Transport der Gebinde 9;91,92,93 innerhalb der Eingangsstation 3 eine erste Fördereinrichtung 34 dient; während b) eine zweite Fördereinrichtung 35 zum Heranschwenken der Gebinde 9;91,92,93 mit an der jeweiligen Passage 47,48 angepressten Abdeckung 98 bestimmt ist; und c) die zweite Fördereinrichtung 35 auch zum Entfernen bearbeiteter Gebinde 9;91,92,93 von der jeweiligen Passage 47,48 nutzbar ist.

Bei einer elften bevorzugten Ausgestaltung der Schleusenanordnung kann zusätzlich zur zehnten bevorzugten Ausgestaltung vorgesehen sein, dass zur Positionierung der Gebinde 9;91,92,93 an der jeweiligen Passage 47,48 die zweite Fördereinrichtung 35 ein kardanisches Lager 351 umfasst.

Bei einer zwölften bevorzugten Ausgestaltung der Schleusenanordnung kann alternativ oder zusätzlich zur zehnten oder elften bevorzugten Ausgestaltung vorgesehen sein, dass die zweite Fördereinrichtung 35 besteht aus: a) einer Achse 350 , durch welche sich eine erste Drehachse D1 erstreckt; b) zwei nebeneinander an der Achse 350 fixierten Gabeln 352 , die jeweils um eine zweite Drehachse D2 schwenkbar sind, welche sich rechtwinklig zur ersten Drehachse D1 erstrecken; und c) jeweils einen in der zugehörigen Gabel 352 aufgehängten und um eine dritte Drehachse D3 schwenkbaren Träger 353 , wobei die erste Drehachse D1 und die dritte Drehachse D3 zueinander parallel verlaufen und die Träger 353 zum Unterfassen der Ränder 941 der Behältnisse 94 beim An- bzw. Abschwenken von den Passagen 47,48 konfiguriert sind; wobei d) zwei nebeneinander liegende kardanische Lager 351 jeweils gebildet sind aus einen U-förmigen Träger 353 , der in der dritten Drehachse D3 jeweils mit einer Gabel 352 verbunden ist, die in der zweiten Drehachse D2 aufgehängt ist.

Bei einer dreizehnten bevorzugten Ausgestaltung der Schleusenanordnung kann alternativ oder zusätzlich zur ersten bis zwölften bevorzugten Ausgestaltung vorgesehen sein, dass in jeder Passage 47,48 eine Dichtung 470,480 sitzt, welche zusammen mit der Abdeckung 98 eines temporär angepressten Gebindes 9;91,92,93 und/oder mit einem temporär angepressten Deckel 51 an den Passagen 47,48 , im Übergang von der Eingangsstation 3 zur Prozesskammer 43 , Abdichtungen schafft.

Bei einer vierzehnten bevorzugten Ausgestaltung der Schleusenanordnung kann alternativ oder zusätzlich zur ersten bis dreizehnten bevorzugten Ausgestaltung vorgesehen sein, dass ein schwenkbarer Ausleger 50 einer in der Prozesskammer 43 positionierten Apparatur 5 den Deckel 51 trägt.

Bei einer fünfzehnten bevorzugten Ausgestaltung der Schleusenanordnung kann alternativ oder zusätzlich zur dritten bis vierzehnten bevorzugten Ausgestaltung vorgesehen sein, der zumindest eine Manipulator 6 oder auch der zusätzliche Manipulator 6 als Roboter ausgebildet sind.

Bei einer sechzehnten bevorzugten Ausgestaltung der Schleusenanordnung kann alternativ oder zusätzlich zur ersten bis fünfzehnten bevorzugten Ausgestaltung vorgesehen sein, dass die Behältnisse 94 der Gebinde 9;91,92,93 die Gestalt wannenförmiger Tubs haben und die Artikel 960 , z.B. Phiolen, Vials oder Spritzen sind, die in systematisch angeordneten Aufnahmekonturen von Nestern 96 stecken.

Es wird ein Verfahren zum kontaminationsfreien Einschleusen eines sterilisierten Objektes aus einem mit einer Abdeckung 98 verschlossenen Behältnis 94 in eine von einer Wandung umgebenen Prozesskammer eines Containments vorgeschlagen, gekennzeichnet durch die folgenden Schritte: eine Passage des Containments wird einerseits von einem in der Prozesskammer herangeführten Deckel und andererseits von einem in der Eingangsstation herangeführten Gebinde mit dessen der Passage zugewandter Abdeckung 98 verschlossen, bei einem an die Passage herangeführtem Gebinde wird eine der Passage zugewandte Oberfläche der Abdeckung mittels einer Dekontaminationsvorrichtung dekontaminiert, nach erfolgter Dekontamination der der Passage zugewandten Oberfläche der Abdeckung 98, einschließlich deren Umfeld, wird die Abdeckung 98 geöffnet, um das Objekt durch die Passage in die Prozesskammer zu transferieren, nach einem Entleeren des Behältnisses wird der Deckel wieder an die Passage herangebracht, um anschließend das von den Objekten geleerte Behältnis von der Passage zu entfernen.

## Patentansprüche

1. Verfahren zum kontaminationsfreien Einschleusen eines sterilisierten Objektes aus einem mit einer Abdeckung (98) verschlossenen Behältnis (94) in eine von einer Wandung umgebenen Prozesskammer eines Containments, **gekennzeichnet durch** die folgenden Schritte:
- eine Passage des Containments wird einerseits von einem in der Prozesskammer herangeführten Deckel und andererseits von einem in der Eingangsstation herangeführten Gebinde mit dessen der Passage zugewandter Abdeckung (98) verschlossen,
- bei einem an die Passage herangeführtem Gebinde wird eine der Passage zugewandte Oberfläche der Abdeckung mittels einer Dekontaminationsvorrichtung dekontaminiert,
- nach erfolgter Dekontamination der der Passage zugewandten Oberfläche der Abdeckung (98), einschließlich deren Umfeld, wird die Abdeckung (98) geöffnet, um das Objekt durch die Passage in die Prozesskammer zu transferieren,
- nach einem Entleeren des Behältnisses wird der Deckel wieder an die Passage herangebracht, um anschließend das von den Objekten geleerte Behältnis von der Passage zu entfernen.

2. Verfahren nach Anspruch 1, **dadurch gekennzeichnet, dass** die Dekontaminationseinrichtung im Deckel integriert ist.

3. Verfahren nach einem der vorangehenden Ansprüche, **dadurch gekennzeichnet, dass** die Abdeckung (98) mit einem in der Prozesskammer stationierten Manipulators aufgeschnitten wird.

4. Verfahren nach einem der vorangehenden Ansprüche, **dadurch gekennzeichnet, dass** die Abdeckung (98) auf einem den Durchlass (95) des Behältnisses (94) umlaufenden Rand (941) versiegelt ist.

5. Verfahren nach einem der vorangehenden Ansprüche, **dadurch gekennzeichnet, dass** ein Träger (353) den Rand (941) des Behältnisses (94) bei einem An- und Abschwenken unterfasst.

6. Verfahren nach einem der vorangehenden Ansprüche, **dadurch gekennzeichnet, dass** das verschlossene Behältnis mit seiner Abdeckung (98) zum Verschließen der Passage (47) an diese angedrückt wird.

7. Verfahren nach einem der vorangehenden Ansprüche, **dadurch gekennzeichnet, dass** in der Passage (47) eine Dichtung (470) sitzt, welche zusammen mit der Abdeckung und/oder mit dem temporär angepressten Deckel Abdichtungen schafft.

8. Verfahren nach einem der vorangehenden Ansprüche, **dadurch gekennzeichnet, dass** das Behältnis ein wannenförmiges Tub ist.

9. Verfahren nach einem der vorangehenden Ansprüche, **dadurch gekennzeichnet, dass** die Abdeckung (98) aus einem semipermeablen Vliesgewebe besteht.

10. Verfahren nach einem der vorangehenden Ansprüche, **dadurch gekennzeichnet, dass** das Behältnis (94) während eines Anpressens auf seiner Rückseite freiliegend bliebt und/oder von einer Laminarströmung (LF) angeströmt wird.

11. Verfahren nach einem der vorangehenden Ansprüche, **dadurch gekennzeichnet, dass** eine Fördereinrichtung keine Dichtung aufweist.

12. Verfahren nach einem der vorangehenden Ansprüche, **dadurch gekennzeichnet, dass** die Fördereinrichtung und/oder eine Gabel, insbesondere während einer Bewegung, von Luft angeströmt wird.

13. Verfahren nach einem der vorangehenden Ansprüche, **dadurch gekennzeichnet, dass** der Deckel an einem Ausleger schwenkbar gelagert ist.

14. Verfahren zum Einschleusen von Artikeln (960) unter aseptischen Bedingungen aus seriell zugeführten Gebinden (9;91,92,93) in eine von einem Gehäuse (49) umgebene Prozesskammer (43) eines Containments (4) mittels einer in einem Aufstellraum (A) positionierten Schleusenanordnung (1), wobei: a) der Prozesskammer (43) eine Eingangsstation (3) vorgelagert ist; b) ein einzelnes Gebinde (9; 91, 92, 93) umfasst: ba) ein Behältnis (94) mit einem aseptischen Innenraum (940) und einem Durchlass (95), der mit einer Abdeckung (98) verschlossen ist; und bb) die im Innenraum (940) gelagerten Artikel (960), die nach Öffnen der Abdeckung (98) in der Prozesskammer (43) zu behandeln sind, **gekennzeichnet durch** die im Produktionsmodus der Schleusenanordnung (1) aufeinanderfolgenden Verfahrensschritte: c) zum Transfer der Artikel (960) aus den Gebinden (9;91,92,93) in die Prozesskammer (43) durch von der Eingangsstation (3) zumindest zwei in die Prozesskammer (43) führende Passagen (47,48) vorgesehen sind und jede der Passagen (47,48) wir einerseits von einem in der Prozesskammer (43) herangeführten Deckel (51) und/oder andererseits von einem in der Eingangsstation (3) herangeführten Gebinde (9;91,92,93) mit dessen der Passage (47,48) zugewandten Abdeckung (98) verschlossen; d) bei an die Passage (47,48) herangeführtem Gebinde (9;91,92,93) wird die der Passage (47,48) zugewandte Oberfläche der Abdeckung (98) mit eingeschlossenem Umfeld (8) mittels einer Dekontaminationsvorrichtung (7) dekontaminiert; e) nach erfolgter Dekontamination der der Passage (47,48) zugewandten Oberfläche der Abdeckung (98), einschliesslich deren Umfeld (8), wird die Abdeckung (98) geöffnet, um die Artikel (960) aus dem Behältnis (94) durch dessen Durchlass (95) in die Prozesskammer (43) zu transferieren; und f) nach Entleerung des Behältnisses (94) wird zunächst der Deckel (51) wieder an die betreffende Passage (47,48) herangebracht, um anschliessend das von den Artikeln (960) geleerte Behältnis (94) von der Passage (47,48) zu entfernen; wobei g) der Deckel (51) an den Passagen (47,48) im Wechsel zum Einsatz kommt, insbesondere unter Ausführung eines Verfahrens nach einem der vorangehenden Ansprüche.

15. Schleusenanordnung zum Einschleusen von Artikeln (960) unter aseptischen Bedingungen aus seriell zugeführten Gebinden (9;91,92,93) in eine von einem Gehäuse (49) umgebene Prozesskammer (43) eines Containments (4) mittels einer in einem Aufstellraum (A) positionierten Schleusenanordnung (1), wobei: a) der Prozesskammer (43) eine Eingangsstation (3) vorgelagert ist; b) ein einzelnes Gebinde (9; 91, 92, 93) umfasst: ba) ein Behältnis (94) mit einem aseptischen Innenraum (940) und einem Durchlass (95), der mit einer Abdeckung (98) verschlossen ist; und bb) die im Innenraum (940) gelagerten Artikel (960), die nach Öffnen der Abdeckung (98) in der Prozesskammer (43) zu behandeln sind, **dadurch gekennzeichnet, dass**: c) zum Transfer der Artikel (960) aus den Gebinden (9;91,92,93) in die Prozesskammer (43) durch von der Eingangsstation (3) zumindest zwei in die Prozesskammer (43) führende Passagen (47,48) vorgesehen sind und jede der Passagen (47,48) einerseits von einem in der Prozesskammer (43) herangeführten Deckel (51) und/oder andererseits von einem in der Eingangsstation (3) herangeführten Gebinde (9;91,92,93) mit dessen der Passage (47,48) zugewandten Abdeckung (98) verschliessbar ist; d) bei an die Passage (47,48) herangeführtem Gebinde (9;91,92,93) die der Passage (47,48) zugewandte Oberfläche der Abdeckung (98) mit eingeschlossenem Umfeld (8) mittels einer Dekontaminationsvorrichtung (7) dekontaminierbar ist; e) nach erfolgter Dekontamination der der Passage (47,48) zugewandten Oberfläche der Abdeckung (98), einschliesslich deren Umfeld (8), sich die Abdeckung (98) öffnen lässt und die Artikel (960) aus dem Behältnis (94) durch dessen Durchlass (95) in die Prozesskammer (43) transferierbar sind; und f) der Deckel (51) wieder an die betreffende Passage (47,48), an welcher das geleerte Behältnis (94) ansteht, anfügbar ist und sich somit das leere Behältnis (94) von der Passage (47,48) entfernen lässt; wobei g) der Deckel (51) im Wechsel zwischen den Passagen (47,48) zum Einsatz kommt.
